# EUROPEAN PATENT SPECIFICATION

(11) **EP 2 991 666 B1**
(45) Date of publication and mention of the grant of the patent: **25.03.2020**
(21) Application number: 14792116.7
(22) Date of filing: 01.05.2014
(51) Int. Cl.: A61K 38/14, C12N 9/10, C12P 21/00, C12Q 1/48, C07K 16/00

(54) **SIALYLATED GLYCOPROTEINS**
SIALYLIERTE GLYCOPROTEINE
GLYCOPROTÉINES SIALYLÉES

(30) Priority: 02.05.2013 US 201361818563 P
(43) Date of publication of application: 09.03.2016
(60) Divisional application: 20158041.2
(73) Proprietor: Momenta Pharmaceuticals, Inc., Cambridge, MA 02142 (US)
(72) Inventor: BHATNAGAR, Naveen, Framingham, MA 01701 (US); MECCARIELLO, Robin, Brighton, MA 02135 (US); LANSING, Jonathan, C., Reading, MA 01867 (US); ORTIZ, Daniel, Stoneham MA 02180-1120 (US); SARVAIYA, Hetal, Quincy, MA 02169 (US); WASHBURN, Nathaniel, Littleton, MA 01460 (US)
(74) Representative: Mathys & Squire
(86) International application number: PCT/US2014/036413
(87) International publication number: WO 2014/179601

(56) References cited:
- EP-A1- 2 233 502
- WO-A1-2012/113863
- WO-A1-2015/057622
- WO-A2-2014/052360
- CA-A1- 2 828 905
- US-A1- 2004 137 106
- US-A1- 2010 113 294
- US-A1- 2011 263 828
- US-B1- 8 278 072
- David H Joziasse ET AL: "1987 by The American Society of Biological Chemists Branch Specificity of Bovine Colostrum CMP-Sialic Acid: Gal@1+4GlcNAc-R cu2+6-Sialyltransferase", THE JOURNAL OF BIOLOGICAL CHEMISTRY 0, 15 February 1987 (1987-02-15), pages 2025-2033, XP055290339, Retrieved from the Internet: URL:http://www.jbc.org/content/262/5/2025. full.pdf [retrieved on 2016-07-21]
- JOZIASSE, D ET AL.: 'Branch Specificity Of Bovine Colostrum CMP-Sialic Acid: Gal beta 1->4GIcNAc-R alpha 2->6-Sialyltransferase: Sialylation Of Bi-, Tri-, And Tetraantennary Oligosaccharides And Glycopeptides Of The N-Acetyllactosamine Type.' J BIOL CHEM. vol. 262, no. 5, 15 February 1987, pages 2025 - 2033, XP055290339
- LANCE, P ET AL.: 'Isolation And Characterization Of A Partial cDNA For A Human Sialyltransferase.' BIOCHEM BIOPHYS RES COMMUN. vol. 164, no. 1, 16 October 1989, pages 225 - 232, XP024839127
- Wei Huang ET AL: "Chemoenzymatic Glycoengineering of Intact IgG Antibodies for Gain of Functions", Journal of the American Chemical Society, vol. 134, no. 29, 16 July 2012 (2012-07-16), pages 12308-12318, XP055531856, ISSN: 0002-7863, DOI: 10.1021/ja3051266

## Description

### FIELD

The present disclosure relates generally to glycobiology and glycoproteins.

### BACKGROUND

Therapeutic glycoproteins are an important class of therapeutic biotechnology products, and therapeutic Fc containing glycoproteins, such as IVIG, Fc-receptor fusions, and antibodies (including murine, chimeric, humanized and human antibodies and fragments thereof) account for the majority of therapeutic biologic products.

WO 2012/113863 A1 discloses a method for producing sialylated IgG antibodies.

US 2011/263828 A1 discloses methods for modifying human antibodies by glycan engineering.

Joziasse et al. (Journal of Biological Chemistry, 1987, 262, 5, 2025-2033) describe the branch specificity of bovine colostrum CMP-sialic acid.

Huang et al. (Journal of the American Chemical Society, 2012, 134, 29, 12308-12318) describe a chemoenzymatic method for glycoengineering of intact IgG antibodies.

### SUMMARY

The present invention provides methods as defined by the appended claims.

More generally, the present disclosure encompasses the discovery of a novel mechanism of sialylation by a sialyltransferase (ST6 Gal-I), which sialylates a substrate (e.g., an Fc-containing glycoprotein comprising branched glycans comprising an α1,3 arm and an α1,6 arm) in an ordered fashion. Specifically, under certain conditions, ST6 sialyltransferase catalyzes addition of a sialic acid on an α1,3 arm, followed by addition of a second sialic acid on an α1,6 arm, followed by removal of sialic acid from an α1,3 arm. Accordingly, activity of ST6 sialyltransferase can be controlled using methods described herein to produce glycoproteins having particular branch sialylation patterns.

In one aspect, the disclosure features a method of producing a preparation of glycoproteins comprising Fc regions comprising branched glycans comprising an α1,3 arm and an α1,6 arm, the preparation comprising (i) a target level of branched glycans having a sialic acid on an α1,3 arm (e.g., with a NeuAc-α2,6-Gal terminal linkage) and/or (ii) a target level of branched glycans having a sialic acid on an α1,6 arm (e.g., with a NeuAc-α2,6-Gal terminal linkage), the method comprising: providing a plurality of glycoproteins comprising Fc regions comprising branched glycans comprising an α1,3 arm and an α1,6 arm; and contacting the glycoproteins with an ST6 sialyltransferase in the presence of a limited reaction condition, thereby producing a glycoprotein preparation having (i) the target level of branched glycans having a sialic acid on the α1,3 arm (e.g., with a NeuAc-α2,6-Gal terminal linkage) and/or (ii) the target level of branched glycans having a sialic acid on an α1,6 arm (e.g., with a NeuAc-α2,6-Gal terminal linkage).

In some parts of the disclosure, the ST6 sialyltransferase has at least 70%, 75%, 80%, 85%, 90%, 91%, 92%, 93%, 94%, 95%, 96%, 97%, 98%, or 99% identity, or is 100% identical, to amino acid residues 95-416 of SEQ ID NO:1, to SEQ ID NO:2, or to SEQ ID NO:3.

In some parts of the disclosure, the limited reaction condition is sufficient for the ST6 sialyltransferase substantially to add a sialic acid to an α1,3 arm of a branched glycan and not sufficient for the ST6 sialyltransferase substantially to add a sialic acid to an α1,6 arm of a branched glycan.

In some embodiments, the method further comprises isolating the glycoprotein preparation. In some embodiments, the method further comprises measuring a level of branched glycans comprising a sialic acid on an α1,3 arm and/or measuring a level of branched glycans having a sialic acid on an α1,6 arm.

In some parts of the disclosure, the level of branched glycans comprising a sialic acid on an α1,3 arm and/or level of branched glycans having a sialic acid on an α1,6 arm is measured by one or more of: releasing glycans (e.g., enzymatically releasing glycans) from glycoproteins and measuring the released glycans; measuring glycans on glycoproteins; derivatizing glycans and measuring derivatized glycans; measuring by fluorescence; measuring by mass spectrometry; and measuring by nuclear magnetic resonance.

In some parts of the disclosure, the target level of branched glycans having a sialic acid on an α1,3 arm is at least 20%, 25%, 30%, 35%, 40%, 45%, 50%, 55%, 60%, 65%, 70%, 75%, 80%, 85%, 90%, 95%, or 100% of glycans, branched glycans, or sialylated branched glycans. In some embodiments, the target level of branched glycans having a sialic acid on an α1,3 arm is less than 100%, 95%, 90%, 80%, 75%, 70%, 70%, 65%, 60%, 55%, 50%, 45%, 40%, 35%, 30%, 25%, 20%, 15%, 10%, or 5% of glycans, branched glycans, or sialylated branched glycans.

In some parts of the disclosure, the target level of branched glycans having a sialic acid on an α1,6 arm is less than 50%, 45%, 40%, 35%, 30%, 25%, 20%, 15%, 10%, 5%, or less of glycans, branched glycans, or sialylated branched glycans. In some parts of the disclosure, the target level of branched glycans having a sialic acid on an α1,6 arm is at least 20%, 25%, 30%, 35%, 40%, 45%, 50%, 55%, 60%, 65%, 70%, 75%, 80%, 85%, 90%, 95%, or 100% of glycans, branched glycans, or sialylated branched glycans.

In some embodiments, target level is a mole percentage, mass percentage, and/or area percentage.

In some parts of the disclosure, the limited reaction condition is selected using a method comprising: a) contacting the glycoproteins with an ST6 sialyltransferase in the presence of a first reaction condition; b) measuring a first level of branched glycans comprising a sialic acid on an α1,3 arm and/or branched glycans comprising a sialic acid on an α1,6 arm after the first reaction condition; c) contacting the glycoproteins with the ST6 sialyltransferase in the presence of a second reaction condition; and d) measuring a second level of branched glycans comprising a sialic acid on an α1,3 arm and/or branched glycans comprising a sialic acid on an α1,6 arm after the second reaction condition; wherein the first reaction condition is selected as the limited reaction condition if the first level of branched glycans comprising a sialic acid on an α1,3 arm is higher than the second level of branched glycans comprising a sialic acid on an α1,3 arm; and/or the first level of branched glycans comprising a sialic acid on an α1,6 arm is lower than the second level of branched glycans comprising a sialic acid on an α1,6 arm. In some embodiments, the first reaction condition is selected from one or more of: a shorter reaction time relative to the second reaction condition; a lower ST6 sialyltransferase concentration and/or specific activity relative to the second reaction condition; a lower temperature relative to the second reaction condition; and a lower concentration of a sialic acid donor relative to the second reaction condition.

In some parts of the disclosure, the limited reaction condition is selected from one or more of: a shorter reaction time relative a control reaction condition; a lower ST6 sialyltransferase concentration and/or specific activity relative to a control reaction condition; a lower temperature relative to a control reaction condition; and a lower concentration of a sialic acid donor relative to a control reaction condition.

In another aspect, the disclosure features a method of producing a preparation of glycoproteins comprising Fc regions comprising branched glycans comprising an α1,3 arm and an α1,6 arm, the preparation comprising (i) a target level of branched glycans having a sialic acid on an α1,6 arm (e.g., with a NeuAc-α2,6-Gal terminal linkage) and/or (ii) a target level of branched glycans having a sialic acid on an α1,3 arm (e.g., with a NeuAc-α2,6-Gal terminal linkage), the method comprising: providing a plurality of glycoproteins comprising Fc regions comprising branched glycans comprising an α1,3 arm and an α1,6 arm; and contacting the glycoproteins with an ST6 sialyltransferase in the presence of an extended reaction condition, thereby producing a glycoprotein preparation having (i) the target level of branched glycans having a sialic acid on the α1,6 arm (e.g., with a NeuAc-α2,6-Gal terminal linkage) and/or (ii) the target level of branched glycans having a sialic acid on an α1,3 arm (e.g., with a NeuAc-α2,6-Gal terminal linkage).

In some embodiments, the extended reaction condition is sufficient for the ST6 sialyltransferase substantially to remove a sialic acid from an α1,3 arm of a disialylated branched glycan comprising a sialic acid on an α1,3 arm and an α1,6 arm.

In some embodiments, the method further comprises isolating the glycoprotein preparation. In some embodiments, the method further comprises measuring a level of branched glycans comprising a sialic acid on an α1,6 arm and/or measuring a level of branched glycans having a sialic acid on an α1,3 arm.

In some parts of the disclosure, level of branched glycans comprising a sialic acid on an α1,6 arm and/or level of branched glycans having a sialic acid on an α1,3 arm is measured by one or more of: releasing glycans (e.g., enzymatically releasing glycans) from glycoproteins and measuring the released glycans; measuring glycans on glycoproteins; derivatizing glycans and measuring derivatized glycans; measuring by fluorescence; measuring by mass spectrometry; and measuring by nuclear magnetic resonance. In some embodiments, target level is a mole percentage, mass percentage, and/or area percentage.

In some parts of the disclosure, the target level of branched glycans having a sialic acid on an α1,6 arm is at least 20%, 25%, 30%, 35%, 40%, 45%, 50%, 55%, 60%, 65%, 70%, 75%, 80%, 85%, 90%, 95%, or 100% of glycans, branched glycans, or sialylated branched glycans. In some parts of the disclosure, the target level of branched glycans having a sialic acid on an α1,6 arm is less than 100%, 95%, 90%, 80%, 75%, 70%, 70%, 65%, 60%, 55%, 50%, 45%, 40%, 35%, 30%, 25%, 20%, 15%, 10%, or 5% of glycans, branced glycans, or sialylated branched glycans.

In some parts of the disclosure, the target level of branched glycans having a sialic acid on an α1,3 arm is less than 50%, 45%, 40%, 35%, 30%, 25%, 20%, 15%, 10%, 5%, or less of glycans, branched glycans, or sialylated branched glycans. In some parts of the disclosure, the target level of branched glycans having a sialic acid on an α1,3 arm is at least 20%, 25%, 30%, 35%, 40%, 45%, 50%, 55%, 60%, 65%, 70%, 75%, 80%, 85%, 90%, 95%, or 100% of glycans, branched glycans, or sialylated branched glycans.

In some embodiments, target level is a mole percentage, mass percentage, and/or area percentage.

In some parts of the disclosure, the extended reaction condition is selected using a method comprising: a) contacting the glycoproteins with an ST6 sialyltransferase in the presence of a first reaction condition; b) measuring a first level of branched glycans comprising a sialic acid on an α1,6 arm and/or branched glycans comprising a sialic acid on an α1,3 arm after the first reaction condition; c) contacting the glycoproteins with the ST6 sialyltransferase in the presence of a second reaction condition; and d) measuring a second level of branched glycans comprising a sialic acid on an α1,6 arm and/or branched glycans comprising a sialic acid on an α1,3 arm after the second reaction condition; wherein the second reaction condition is selected as the extended reaction condition if the second level of branched glycans comprising a sialic acid on an α1,6 arm is higher than the first level of branched glycans comprising a sialic acid on an α1,6 arm; and/or the second level of branched glycans comprising a sialic acid on an α1,3 arm is lower than the first level of branched glycans comprising a sialic acid on an α1,3 arm. In some parts of the disclosure, the second reaction condition is selected from one or more of: a greater reaction time relative to the first reaction condition; a higher ST6 sialyltransferase concentration and/or specific activity relative to the first reaction condition; a higher temperature relative to the first reaction condition; and a higher concentration of a sialic acid donor relative to the first reaction condition.

In some parts of the disclosure, the extended reaction condition is selected from one or more of: a greater reaction time relative a control reaction condition; a higher ST6 sialyltransferase concentration and/or specific activity relative to a control reaction condition; a higher temperature relative to a control reaction condition; and a higher concentration of a sialic acid donor relative to a control reaction condition.

In another aspect, the disclosure features a method of producing a preparation of glycoproteins comprising Fc regions comprising branched glycans comprising an α1,3 arm and an α1,6 arm, the preparation comprising (i) a target level of disialylated branched glycans having a sialic acid on an α1,3 arm (e.g., with a NeuAc-α2,6-Gal terminal linkage) and on an α1,6 arm (e.g., with a NeuAc-α2,6-Gal terminal linkage), (ii) a target level of monosialylated branched glycans having a sialic acid on an α1,3 arm (e.g., with a NeuAc-α2,6-Gal terminal linkage) and/or (iii) a target level of monosialylated branched glycans having a sialic acid on an α1,6 arm (e.g., with a NeuAc-α2,6-Gal terminal linkage), the method comprising: providing a plurality of glycoproteins comprising Fc regions comprising branched glycans comprising an α1,3 arm and an α1,6 arm; and contacting the glycoproteins with an ST6 sialyltransferase in the presence of an intermediate reaction condition, thereby producing a glycoprotein preparation having (i) the target level of disialylated branched glycans having a sialic acid on the α1,3 arm (e.g., with a NeuAc-α2,6-Gal terminal linkage) and on the α1,6 arm (e.g., with a NeuAc-α2,6-Gal terminal linkage), (ii) the target level of monosialylated branched glycans having a sialic acid on an α1,3 arm (e.g., with a NeuAc-α2,6-Gal terminal linkage), and/or (iii) the target level of monosialylated branched glycans having a sialic acid on an α1,6 arm (e.g., with a NeuAc-α2,6-Gal terminal linkage).

In some parts of the disclosure, the intermediate reaction condition is sufficient for the ST6 sialyltransferase substantially to add a sialic acid to an α1,3 arm and to an α1,6 arm of a branched glycan, and not sufficient for the ST6 sialyltransferase substantially to remove a sialic acid from an α1,3 arm of a branched glycan.

In some embodiments, the method further comprises isolating the glycoprotein preparation. In some embodiments, the method further comprises measuring a level of (i) disialylated branched glycans having a sialic acid on an α1,3 arm and on an α1,6 arm, (ii) monosialylated branched glycans having a sialic acid on an α1,3 arm and/or (iii) monosialylated branched glycans having a sialic acid on an α1,6 arm.

In some parts of the disclosure, level of (i) disialylated branched glycans having a sialic acid on an α1,3 arm and on an α1,6 arm, (ii) monosialylated branched glycans having a sialic acid on an α1,3 arm and/or (iii) monosialylated branched glycans having a sialic acid on an α1,6 arm is measured by one or more of: releasing glycans (e.g., enzymatically releasing glycans) from glycoproteins and measuring the released glycans; measuring glycans on glycoproteins; derivatizing glycans and measuring derivatized glycans; measuring by fluorescence; measuring by mass spectrometry; and measuring by nuclear magnetic resonance.

In some embodiments, the target level of disialylated branched glycans having a sialic acid on an α1,3 arm and on an α1,6 arm is at least 60%, 65%, 70%, 75%, 80%, 85%, 90%, 95%, or 100% of glycans, branched glycans, or sialylated branched glycans. In some parts of the disclosure, the target level of disialylated branched glycans having a sialic acid on an α1,3 arm and on an α1,6 arm is less than 100%, 95%, 90%, 80%, 75%, 70%, 70%, 65%, 60%, 55%, 50%, 45%, 40%, 35%, 30%, 25%, 20%, 15%, 10%, or 5% of glycans, branched glycans, or sialylated branched glycans.

In some parts of the disclosure, the target level of monosialylated branched glycans having a sialic acid on an α1,3 arm is less than 50%, 45%, 40%, 35%, 30%, 25%, 20%, 15%, 10%, 5%, or less of glycans, branched glycans, or sialylated branched glycans. In some parts of the disclosure, the target level of monosialylated branched glycans having a sialic acid on an α1,3 arm is at least 20%, 25%, 30%, 35%, 40%, 45%, 50%, 55%, 60%, 65%, 70%, 75%, 80%, 85%, 90%, 95%, or 100% of glycans, branched glycans, or sialylated branched glycans.

In some parts of the disclosure, the target level of monosialylated branched glycans having a sialic acid on an α1,6 arm is less than 50%, 45%, 40%, 35%, 30%, 25%, 20%, 15%, 10%, 5%, or less of sialylated branched glycans. In some parts of the disclosure, the target level of monosialylated branched glycans having a sialic acid on an α1,6 arm is at least 20%, 25%, 30%, 35%, 40%, 45%, 50%, 55%, 60%, 65%, 70%, 75%, 80%, 85%, 90%, 95%, or 100% of glycans, branched glycans, or sialylated branched glycans.

In some embodiments, target level is a mole percentage, mass percentage, and/or area percentage.

In another aspect, the disclosure features a method of producing a preparation of glycoproteins comprising Fc regions comprising branched glycans comprising an α1,3 arm and an α1,6 arm, the preparation comprising (i) a target level of branched glycans having a sialic acid on an α1,6 arm (e.g., with a NeuAc-α2,6-Gal terminal linkage) and/or (ii) a target level of branched glycans having a sialic acid on an α1,3 arm (e.g., with a NeuAc-α2,6-Gal terminal linkage), the method comprising: providing a plurality of glycoproteins comprising Fc regions comprising branched glycans comprising an α1,3 arm and an α1,6 arm; and contacting the glycoproteins with an ST6 sialyltransferase in the presence of an initial reaction condition sufficient for the ST6 sialyltransferase substantially to add a sialic acid to an α1,3 arm (e.g., with a NeuAc-α2,6-Gal terminal linkage) and to add a sialic acid to an α1,6 arm (e.g., with a NeuAc-α2,6-Gal terminal linkage) of a branched glycan to produce a disialylated branched glycan; and contacting the disialylated branched glycan with the ST6 sialyltransferase in the presence of an extended reaction condition, thereby producing a glycoprotein preparation having (i) the target level of branched glycans having a sialic acid on the α1,6 arm (e.g., with a NeuAc-α2,6-Gal terminal linkage) and/or (ii) the target level of branched glycans having a sialic acid on an α1,3 arm (e.g., with a NeuAc-α2,6-Gal terminal linkage).

In another aspect, the disclosure features a method of producing a preparation of glycoproteins comprising Fc regions comprising branched glycans comprising an α1,3 arm and an α1,6 arm, the preparation comprising (i) a target level of disialylated branched glycans having a sialic acid on an α1,3 arm (e.g., with a NeuAc-α2,6-Gal terminal linkage) and on an α1,6 arm (e.g., with a NeuAc-α2,6-Gal terminal linkage), (ii) a target level of monosialylated branched glycans having a sialic acid on an α1,3 arm (e.g., with a NeuAc-α2,6-Gal terminal linkage) and/or (iii) a target level of monosialylated branched glycans having a sialic acid on an α1,6 arm (e.g., with a NeuAc-α2,6-Gal terminal linkage), the method comprising: providing a plurality of glycoproteins comprising Fc regions comprising branched glycans comprising an α1,3 arm and an α1,6 arm; and contacting the glycoproteins with an ST6 sialyltransferase in the presence of an initial reaction condition sufficient for the ST6 sialyltransferase substantially to add a sialic acid to an α1,3 arm (e.g., with a NeuAc-α2,6-Gal terminal linkage) of a branched glycan to produce a monosialylated branched glycan; and contacting the monosialylated branched glycan with the ST6 sialyltransferase in the presence of an extended reaction condition, thereby producing a glycoprotein preparation having (i) the target level of disialylated branched glycans having a sialic acid on an α1,3 arm (e.g., with a NeuAc-α2,6-Gal terminal linkage) and on an α1,6 arm (e.g., with a NeuAc-α2,6-Gal terminal linkage), (ii) the target level of monosialylated branched glycans having a sialic acid on an α1,3 arm (e.g., with a NeuAc-α2,6-Gal terminal linkage) and/or (iii) the target level of monosialylated branched glycans having a sialic acid on an α1,6 arm (e.g., with a NeuAc-α2,6-Gal terminal linkage).

In another aspect, the disclosure features a method of removing a sialic acid from a branched glycan of an Fc region, the branched glycan comprising an α1,3 arm and an α1,6 arm, the method comprising: providing a branched glycan of an Fc region, the branched glycan comprising an α1,3 arm and an α1,6 arm and comprising a sialic acid on the α1,3 arm (e.g., with a NeuAc-α2,6-Gal terminal linkage); contacting the branched glycan with an ST6 sialyltransferase in the presence of an initial reaction condition sufficient for the ST6 sialyltransferase to add a sialic acid to the α1,6 arm (e.g., with a NeuAc-α2,6-Gal terminal linkage) to produce a disialylated branched glycan; and contacting the disialylated branched glycan with the ST6 sialyltransferase in the presence of an extended reaction condition, thereby removing the sialic acid from the α1,3 arm of the branched glycan.

In another aspect, the disclosure features a method of modulating sialylation of Fc region branched glycans comprising an α1,3 arm and an α1,6 arm, the method comprising: providing a reaction solution comprising (i) Fc region branched glycans comprising an α1,3 arm and an α1,6 arm, (ii) a ST6 sialyltransferase, and (iii) a sialic acid donor; and incubating the reaction solution under reaction conditions sufficient for the ST6 sialyltransferase to catalyze transfer of a sialic acid primarily to the α1,3 arm (e.g., with a NeuAc-α2,6-Gal terminal linkage) only, primarily to the α1,6 arm (e.g., with a NeuAc-α2,6-Gal terminal linkage) only, or to both the α1,3 arm (e.g., with a NeuAc-α2,6-Gal terminal linkage) and the α1,6 arm (e.g., with a NeuAc-α2,6-Gal terminal linkage), wherein: a) incubating the reaction solution under reaction conditions sufficient for the sialyltransferase to catalyze transfer of the sialic acid primarily to the α1,3 arm comprises controlling reaction kinetics such that: (i) the sialic acid addition rate for the α1,3 arm (Rₐ^{1,3}) exceeds the sialic acid addition rate for the α1,6 arm (Rₐ^{1,6}); or (ii) the sialic acid removal rate for the α1,6 arm (Rᵣ^{1,6}) exceeds Rₐ^{1,6}; b) incubating the reaction solution under reaction conditions sufficient for the sialyltransferase to catalyze transfer of the sialic acid primarily to the α1,6 arm comprises controlling reaction kinetics such that: (i) Rₐ^{1,6} exceeds Rᵣ^{1,6}; and (ii) the sialic acid removal rate for the α1,3 arm (Rᵣ^{1,3}) eventually exceeds Rₐ^{1,3}; or c) incubating the reaction solution under reaction conditions sufficient for the sialyltransferase to catalyze transfer of the sialic acid to both the α1,3 and α1,6 arms comprises controlling reaction kinetics such that: (i) Rₐ^{1,3} exceeds Rᵣ^{1,3} ; and (ii) Rₐ^{1,6} exceeds Rᵣ^{1,6}; thereby modulating sialylation of a branched glycan.

In some parts of the disclosure, controlling reaction kinetics comprises one or more of: modulating (e.g., increasing or decreasing) the time of the reaction; modulating (e.g., increasing or decreasing) level or activity of the sialyltransferase; and modulating (e.g., increasing or decreasing) the Rᵣ^{1,3} or Rᵣ^{1,6} rates by controlling or adjusting the ratio of the sialic acid donor to a sialic acid donor reaction product.

In some parts of the disclosure, the sialic acid donor is cytidine 5'-monophospho-N-acetyl neuraminic acid and the sialic acid donor reaction product is cytidine 5'-monophosphate.

In some embodiments, the reaction conditions sufficient for the sialyltransferase to catalyze transfer of the sialic acid to both the α1,3 and α1,6 arms comprises supplementing the sialic donor at least once during the reaction. In some parts of the disclosure, the reaction conditions sufficient for the sialyltransferase to catalyze transfer of the sialic acid to both the α1,3 and α1,6 arms comprises removing a sialic donor reaction product at least once during the reaction. In some embodiments, the reaction conditions sufficient for the sialyltransferase to catalyze transfer of the sialic acid to both the α1,3 and α1,6 arms comprises supplementing the sialic donor reaction product at least once during the reaction.

In some embodiments, the method further comprises detecting reaction kinetics.

In some embodiments, the method further comprises measuring a level of sialylated glycans (e.g., a level of disialylated branched glycans having a sialic acid on an α1,3 arm and on an α1,6 arm, (ii) a level of monosialylated branched glycans having a sialic acid on an α1,3 arm and/or (iii) a level of monosialylated branched glycans having a sialic acid on an α1,6 arm). In some parts of the disclosure, level of sialylated glycans is measured by one or more of: releasing glycans (e.g., enzymatically releasing glycans) from glycoproteins and measuring the released glycans; measuring glycans on glycoproteins; derivatizing glycans and measuring derivatized glycans; measuring by fluorescence; measuring by mass spectrometry; and measuring by nuclear magnetic resonance.

In some parts of the disclosure, the Fc region branched glycans are on, or are derived from, a glycoprotein preparation. In some embodiments, the method further comprises formulating the preparation into a drug product if the preparation meets a target level, e.g., a target level described herein.

In another aspect, the disclosure features a method of producing a preparation of glycoproteins comprising Fc regions comprising branched glycans comprising an α1,3 arm and an α1,6 arm, the preparation comprising (i) a target level of branched glycans having a sialic acid on an α1,3 arm and/or (ii) a target level of branched glycans having a sialic acid on an α1,6 arm, the method comprising: providing a plurality of glycoproteins comprising Fc regions comprising branched glycans comprising an α1,3 arm and an α1,6 arm; contacting the glycoproteins with an ST6 sialyltransferase in the presence of a limited reaction condition sufficient for the ST6 sialyltransferase substantially to add a sialic acid to an α1,3 arm of a branched glycan and not sufficient for the ST6 sialyltransferase substantially to add a sialic acid to an α1,6 arm of a branched glycan, thereby producing a preparation of sialylated glycoproteins; and processing (e.g., one or more of formulating, filling into a container, labeling, packaging) the preparation into a drug product if the preparation meets the target level of branched glycans having a sialic acid on the α1,3 arm and/or the target level of branched glycans having a sialic acid on an α1,6 arm.

In another aspect, the disclosure features a method of producing a preparation of glycoproteins comprising Fc regions comprising branched glycans comprising an α1,3 arm and an α1,6 arm, the preparation comprising (i) a target level of branched glycans having a sialic acid on an α1,6 arm and/or (ii) a target level of branched glycans having a sialic acid on an α1,3 arm, the method comprising: providing a plurality of glycoproteins comprising Fc regions comprising branched glycans comprising an α1,3 arm and an α1,6 arm; contacting the glycoproteins with an ST6 sialyltransferase in the presence of an extended reaction condition sufficient for the ST6 sialyltransferase substantially to remove a sialic acid from an α1,3 arm of a disialylated branched glycan comprising a sialic acid on an α1,3 arm and an α1,6 arm, thereby producing a preparation of sialylated glycoproteins; and processing (e.g., one or more of formulating, filling into a container, labeling, packaging) the preparation into a drug product if the preparation meets the target level of branched glycans having a sialic acid on the α1,6 arm and/or the target level of branched glycans having a sialic acid on an α1,3 arm.

In another aspect, the disclosure features a method of producing a preparation of glycoproteins comprising Fc regions comprising branched glycans comprising an α1,3 arm and an α1,6 arm, the preparation comprising (i) a target level of disialylated branched glycans having a sialic acid on an α1,3 arm and on an α1,6 arm, (ii) a target level of monosialylated branched glycans having a sialic acid on an α1,3 arm and/or (iii) a target level of monosialylated branched glycans having a sialic acid on an α1,6 arm, the method comprising: providing a plurality of glycoproteins comprising Fc regions comprising branched glycans comprising an α1,3 arm and an α1,6 arm; contacting the glycoproteins with an ST6 sialyltransferase in the presence of an intermediate reaction condition sufficient for the ST6 sialyltransferase substantially to add a sialic acid to an α1,3 arm and to an α1,6 arm of a branched glycan, and not sufficient for the ST6 sialyltransferase substantially to remove a sialic acid from an α1,3 arm of a branched glycan, thereby producing a preparation of sialylated glycoproteins; and processing (e.g., one or more of formulating, filling into a container, labeling, packaging) the preparation into a drug product if the preparation meets (i) the target level of disialylated branched glycans having a sialic acid on an α1,3 arm and on an α1,6 arm, (ii) the target level of monosialylated branched glycans having a sialic acid on an α1,3 arm and/or (iii) the target level of monosialylated branched glycans having a sialic acid on an α1,6 arm.

In any of the aspects described herein, in some parts of the disclosure, the target level of sialylated branched glycans (e.g., level of branched glycans having a sialic acid on an α1,3 arm, level of branched glycans having a sialic acid on an α1,6 arm, and/or level of branched glycans having a sialic acid on an α1,3 arm and on an α1,6 arm) is a level of sialylated branched glycans in a reference therapeutic product. In some parts of the disclosure, the target level of sialylated branched glycans is a level in a reference therapeutic antibody product. In some parts of the disclosure, the target level of sialylated glycans is a pharmaceutical product specification or a quality control criterion for a pharmaceutical preparation, e.g., a Certificate of Analysis (CofA), a Certificate of Testing (CofT), or a Master Batch Record. In some parts of the disclosure, the product specification is a product description in an FDA label, a Physician's Insert, a USP monograph, or an EP monograph.

In some parts of the disclosure, the reference therapeutic product is selected from the group consisting of: abatacept, abciximab, adalimumab, aflibercept, alefacept, alemtuzumab, basiliximab, bevacizumab, belatacept, certolizumab, cetuximab, daclizumab, eculizumab, efalizumab, entanercept, gemtuzumab, ibritumomab, infliximab, muromonab-CD3, natalizumab, omalizumab, palivizumab; panitumumab, ranibizumab, rilonacept, rituximab, tositumomab, and trastuzumab.

In any of the aspects described herein, in some embodiments, the preparation is an IVIG preparation. In some parts of the disclosure, the preparation is a recombinant Fc containing glycoprotein preparation. In some parts of the disclosure, the recombinant glycoprotein is a recombinant antibody or Fc fusion protein.

In another aspect, the disclosure features a glycoprotein preparation produced by any of the methods described herein.

### BRIEF DESCRIPTION OF THE DRAWINGS

The present teachings described herein will be more fully understood from the following description of various illustrative embodiments, when read together with the accompanying drawings. It should be understood that the drawings described below are for illustration purposes only and are not intended to limit the scope of the present teachings in any way.
FIG. 1 is a schematic illustration of a common core pentasaccharide (Man)₃(GlcNAc)(GlcNAc) of N-glycans.
FIG. 2 is a schematic illustration of an IgG antibody molecule.
FIG. 3 is a graphic representation of relative abundance of glycans at various times during a sialylation reaction with ST6 sialyltransferase.
FIG. 4 is a schematic illustration of a reaction scheme for ST6 sialyltransferase (fucose: triangles, N-acetylglucosamine: squares, mannose: dark circles, galactose: light circles, sialic acid: diamonds).

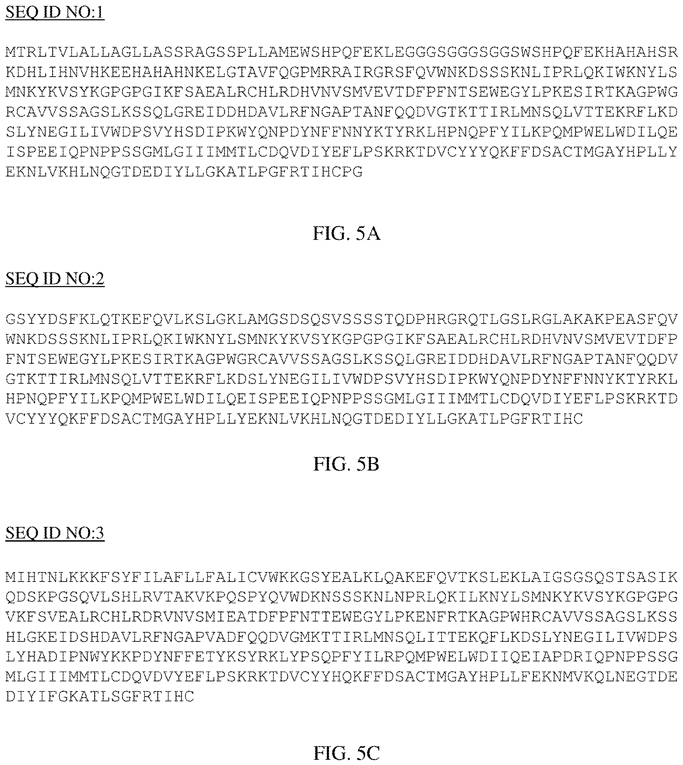

FIG. 5A depicts an exemplary ST6 sialyltransferase amino acid sequence (SEQ ID NO:1). FIG. 5B depicts an exemplary ST6 sialyltransferase amino acid sequence (SEQ ID NO:2). FIG. 5C depicts an exemplary ST6 sialyltransferase amino acid sequence (SEQ ID NO:3).

### DETAILED DESCRIPTION

Antibodies are glycosylated at conserved positions in the constant regions of their heavy chain. For example, IgG antibodies have a single N-linked glycosylation site at Asn297 of the CH2 domain. Each antibody isotype has a distinct variety of N-linked carbohydrate structures in the constant regions. For human IgG, the core oligosaccharide normally consists of GlcNAc₂Man₃GlcNAc, with differing numbers of outer residues. Variation among individual IgG's can occur via attachment of galactose and/or galactose-sialic acid at one or both terminal GlcNAc or via attachment of a third GlcNAc arm (bisecting GlcNAc).

The present disclosure encompasses glycoprotein preparations (e.g., Fc region-containing glycoprotein preparations (e.g., IVIG, Fc or IgG antibodies)) having particular levels of branched glycans that are sialylated on an α1,3 arm, an α1,6 arm, or both, of the branched glycans in the Fc region (e.g., with a NeuAc-α2,6-Gal terminal linkage). The levels can be measured on an individual Fc region (e.g., the number of branched glycans that are sialylated on an α1,3 arm, an α1,6 arm, or both, of the branched glycans in the Fc region), or on the overall composition of a preparation of glycoproteins (e.g., the number or percentage of branched glycans that are sialylated on an α1,3 arm, an α1,6 arm, or both, of the branched glycans in the Fc region in a preparation of glycoproteins).

### Definitions

As used herein, "glycan" is a sugar, which can be monomers or polymers of sugar residues, such as at least three sugars, and can be linear or branched. A "glycan" can include natural sugar residues (e.g., glucose, N-acetylglucosamine, N-acetyl neuraminic acid, galactose, mannose, fucose, hexose, arabinose, ribose, xylose, etc.) and/or modified sugars (e.g., 2'-fluororibose, 2'-deoxyribose, phosphomannose, 6'sulfo N-acetylglucosamine, etc.). The term "glycan" includes homo and heteropolymers of sugar residues. The term "glycan" also encompasses a glycan component of a glycoconjugate (e.g., of a glycoprotein, glycolipid, proteoglycan, etc.). The term also encompasses free glycans, including glycans that have been cleaved or otherwise released from a glycoconjugate.

As used herein, the term "glycoprotein" refers to a protein that contains a peptide backbone covalently linked to one or more sugar moieties (i.e., glycans). The sugar moiety(ies) may be in the form of monosaccharides, disaccharides, oligosaccharides, and/or polysaccharides. The sugar moiety(ies) may comprise a single unbranched chain of sugar residues or may comprise one or more branched chains. Glycoproteins can contain O-linked sugar moieties and/or N-linked sugar moieties.

As used herein, the term "glycoprotein preparation" refers to a set of individual glycoprotein molecules, each of which comprises a polypeptide having a particular amino acid sequence (which amino acid sequence includes at least one glycosylation site) and at least one glycan covalently attached to the at least one glycosylation site. Individual molecules of a particular glycoprotein within a glycoprotein preparation typically have identical amino acid sequences but may differ in the occupancy of the at least one glycosylation sites and/or in the identity of the glycans linked to the at least one glycosylation sites. That is, a glycoprotein preparation may contain only a single glycoform of a particular glycoprotein, but more typically contains a plurality of glycoforms. Different preparations of the same glycoprotein may differ in the identity of glycoforms present (e.g., a glycoform that is present in one preparation may be absent from another) and/or in the relative amounts of different glycoforms.

The term "glycoform" is used herein to refer to a particular form of a glycoprotein. That is, when a glycoprotein includes a particular polypeptide that has the potential to be linked to different glycans or sets of glycans, then each different version of the glycoprotein (i.e., where the polypeptide is linked to a particular glycan or set of glycans) is referred to as a "glycoform".

"Reference glycoprotein", as used herein, refers to a glycoprotein having substantially the same amino acid sequence as (e.g., having about 95-100% identical amino acids of) a glycoprotein described herein, e.g., a glycoprotein to which it is compared. In some embodiments, a reference glycoprotein is a therapeutic glycoprotein described herein, e.g., an FDA approved therapeutic glycoprotein.

As used herein, the term "antibody" refers to a polypeptide that includes at least one immunoglobulin variable region, e.g., an amino acid sequence that provides an immunoglobulin variable domain or immunoglobulin variable domain sequence. For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody" encompasses antigen-binding fragments of antibodies (e.g., single chain antibodies, Fab, F(ab')₂, Fd, Fv, and dAb fragments) as well as complete antibodies, e.g., intact immunoglobulins of types IgA, IgG, IgE, IgD, IgM (as well as subtypes thereof). The light chains of the immunoglobulin can be of types kappa or lambda.

As used herein, the term "Fc region" refers to a dimer of two "Fc polypeptides", each "Fc polypeptide" comprising the constant region of an antibody excluding the first constant region immunoglobulin domain. In some embodiments, an "Fc region" includes two Fc polypeptides linked by one or more disulfide bonds, chemical linkers, or peptide linkers. "Fc polypeptide" refers to the last two constant region immunoglobulin domains of IgA, IgD, and IgG, and the last three constant region immunoglobulin domains of IgE and IgM, and may also include part or all of the flexible hinge N-terminal to these domains. For IgG, "Fc polypeptide" comprises immunoglobulin domains Cgamma2 (Cy2) and Cgamma3 (Cy3) and the lower part of the hinge between Cgamma1 (Cy1) and Cy2. Although the boundaries of the Fc polypeptide may vary, the human IgG heavy chain Fc polypeptide is usually defined to comprise residues starting at T223 or C226 or P230, to its carboxyl-terminus, wherein the numbering is according to the EU index as in Kabat et al. (1991, NIH Publication 91-3242, National Technical Information Services, Springfield, VA). For IgA, Fc polypeptide comprises immunoglobulin domains Calpha2 (Cα2) and Calpha3 (Cα3) and the lower part of the hinge between Calpha1 (Cα1) and Cα2. An Fc region can be synthetic, recombinant, or generated from natural sources such as IVIG.

As used herein, an "N-glycosylation site of an Fc region" refers to an amino acid residue within an Fc region to which a glycan is N-linked.

"Predetermined level" or "target level" as used herein, refers to a pre-specified particular level of one or more particular glycans, e.g., branched glycans having a sialic acid on an α1,3 arm, and/or branched glycans having a sialic acid on an α1,6 arm, and/or branched glycans having a sialic acid on an α1,3 arm and on an α1,6 arm. In some embodiments, a predetermined or target level is an absolute value or range. In some embodiments, a predetermined or target level is a relative value. In some embodiments, a predetermined level is the same as or different (e.g., higher or lower than) a level of one or more particular glycans (e.g., branched glycans having a sialic acid on an α1,3 arm, and/or branched glycans having a sialic acid on an α1,6 arm, and/or branched glycans having a sialic acid on an α1,3 arm and on an α1,6 arm) in a reference, e.g., a reference glycoprotein product, or a reference document such as a specification, alert limit, or master batch record for a pharmaceutical product.

In some embodiments, a predetermined or target level is an absolute level or range of (e.g., number of moles of) one or more glycans (e.g., branched glycans having a sialic acid on an α1,3 arm, and/or branched glycans having a sialic acid on an α1,6 arm, and/or branched glycans having a sialic acid on an α1,3 arm and on an α1,6 arm) in a glycoprotein preparation. In some embodiments, a predetermined or target level is a level or range of one or more glycans (e.g., branched glycans having a sialic acid on an α1,3 arm, and/or branched glycans having a sialic acid on an α1,6 arm, and/or branched glycans having a sialic acid on an α1,3 arm and on an α1,6 arm) in a glycoprotein preparation relative to total level of glycans in the glycoprotein preparation. In some embodiments, a predetermined or target level is a level or range of one or more glycans (e.g., branched glycans having a sialic acid on an α1,3 arm, and/or branched glycans having a sialic acid on an α1,6 arm, and/or branched glycans having a sialic acid on an α1,3 arm and on an α1,6 arm) in a glycoprotein preparation relative to total level of sialylated glycans in the glycoprotein preparation. In some embodiments, a predetermined or target level is expressed as a percent.

For any given parameter, in some embodiments, "percent" refers to the number of moles of a particular glycan (glycan X) relative to total moles of glycans of a preparation. In some embodiments, "percent" refers to the number of moles of PNGase F-released Fc glycan X relative to total moles of PNGase F-released Fc glycans detected.

By "purified" (or "isolated") refers to a nucleic acid sequence (e.g., a polynucleotide) or an amino acid sequence (e.g., a polypeptide) that is removed or separated from other components present in its natural environment. For example, an isolated polypeptide is one that is separated from other components of a cell in which it was produced (e.g., the endoplasmic reticulum or cytoplasmic proteins and RNA). An isolated polynucleotide is one that is separated from other nuclear components (e.g., histones) and/or from upstream or downstream nucleic acid sequences. An isolated nucleic acid sequence or amino acid sequence can be at least 60% free, or at least 75% free, or at least 90% free, or at least 95% free from other components present in natural environment of the indicated nucleic acid sequence or amino acid sequence.

As used herein, "polynucleotide" (or "nucleotide sequence" or "nucleic acid molecule") refers to an oligonucleotide, nucleotide, or polynucleotide, and fragments or portions thereof, and to DNA or RNA of genomic or synthetic origin, which may be single- or double- stranded, and represent the sense or antisense strand.

As used herein, "polypeptide" (or "amino acid sequence" or "protein") refers to an oligopeptide, peptide, polypeptide, or protein sequence, and fragments or portions thereof, and to naturally occurring or synthetic molecules. "Amino acid sequence" and like terms, such as "polypeptide" or "protein", are not meant to limit the indicated amino acid sequence to the complete, native amino acid sequence associated with the recited protein molecule.

The term "pharmaceutically effective amount" or "therapeutically effective amount" refers to an amount (e.g., dose) effective in treating a patient, having a disorder or condition described herein. It is also to be understood herein that a "pharmaceutically effective amount" may be interpreted as an amount giving a desired therapeutic effect, either taken in one dose or in any dosage or route, taken alone or in combination with other therapeutic agents.

The term "treatment" or "treating", as used herein, refers to administering a therapy in an amount, manner, and/or mode effective to improve a condition, symptom, or parameter associated with a disorder or condition or to prevent or reduce progression of a disorder or condition, to a degree detectable to one skilled in the art. An effective amount, manner, or mode can vary depending on the subject and may be tailored to the subject.

As used herein, a "characteristic sequence" is a sequence that is found in all members of a family of polypeptides or nucleic acids, and therefore can be used by those of ordinary skill in the art to define members of the family.

As used herein, the term "homology" refers to the overall relatedness between polymeric molecules, e.g., between nucleic acid molecules (e.g., DNA molecules and/or RNA molecules) and/or between polypeptide molecules. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% identical. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% similar.

As used herein, the term "identity" refers to the overall relatedness between polymeric molecules, e.g., between nucleic acid molecules (e.g., DNA molecules and/or RNA molecules) and/or between polypeptide molecules. Calculation of the percent identity of two nucleic acid sequences, for example, can be performed by aligning the two sequences for optimal comparison purposes (e.g., gaps can be introduced in one or both of a first and a second nucleic acid sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or substantially 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using the algorithm of Meyers and Miller (CABIOS, 1989, 4:11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix.

As used herein, the term "ST6 sialyltransferase" refers to a polypeptide whose amino acid sequence includes at least one characteristic sequence of and/or shows at least 100%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71% or 70% identity with a protein involved in transfer of a sialic acid to a terminal galactose of a glycan through an α2,6 linkage (e.g., ST6 Gal-I). A wide variety of ST6 sialyltransferase sequences are known in the art, such as those described herein; in some parts of the disclosure, an ST6 sialyltransferase shares at least one characteristic sequence of and/or shows the specified degree of overall sequence identity with one of the ST6 sialyltransferases set forth herein (each of which may be considered a "reference" ST6 sialyltransferase). In some parts of the disclosure, an ST6 sialyltransferase as described herein shares at least one biological activity with a reference ST6 sialyltransferase as set forth herein. In some such parts of the disclosure, the shared biological activity relates to transfer of a sialic acid to a glycan.

### Glycoproteins

Described herein are preparations (e.g., therapeutic preparations) of polypeptides (e.g., glycoproteins), and methods of making and using such preparations, having particular levels of branched glycans having sialylation on an α1,3 arm, an α1,6 arm, and/or on both arms. Glycoproteins include, for example, any of a variety of hematologic agents (including, for instance, erythropoietin, blood-clotting factors, etc.), interferons, colony stimulating factors, antibodies, enzymes, and hormones. The identity of a particular glycoprotein is not intended to limit the present disclosure, and a preparation described herein can include any glycoprotein of interest, e.g., a glycoprotein having an Fc region.

A glycoprotein described herein can include a target-binding domain that binds to a target of interest (e.g., binds to an antigen). For example, a glycoprotein, such as an antibody, can bind to a transmembrane polypeptide (e.g., receptor) or ligand (e.g., a growth factor). Exemplary molecular targets (e.g., antigens) for glycoproteins described herein (e.g., antibodies) include CD proteins such as CD2, CD3, CD4, CD8, CD11, CD19, CD20, CD22, CD25, CD33, CD34, CD40, CD52; members of the ErbB receptor family such as the EGF receptor (EGFR, HER1, ErbB1), HER2 (ErbB2), HER3 (ErbB3) or HER4 (ErbB4) receptor; macrophage receptors such as CRIg; tumor necrosis factors such as TNFα or TRAIL/Apo-2; cell adhesion molecules such as LFA-1, Mac1, p150,95, VLA-4, ICAM-1, VCAM and αvβ3 integrin including either α or β subunits thereof (e.g., anti-CD11a, anti-CD18 or anti-CD11b antibodies); growth factors and receptors such as EGF, FGFR (e.g., FGFR3) and VEGF; IgE; cytokines such as IL1; cytokine receptors such as IL2 receptor; blood group antigens; flk2/flt3 receptor; obesity (OB) receptor; mpl receptor; CTLA-4; protein C; neutropilins; ephrins and receptors; netrins and receptors; slit and receptors; chemokines and chemokine receptors such as CCL5, CCR4, CCR5; amyloid beta; complement factors, such as complement factor D; lipoproteins, such as oxidized LDL (oxLDL); lymphotoxins, such as lymphotoxin alpha (LTa). Other molecular targets include Tweak, B7RP-1, proprotein convertase subtilisin/kexin type 9 (PCSK9), sclerostin, c-kit, Tie-2, c-fms, and anti-M1.

### Reference Polypeptides

In some parts of the disclosure, methods described herein are useful for controlling the sialylation of a reference polypeptide (e.g., a reference glycoprotein). In some parts of the disclosure, polypeptide (e.g., glycoprotein) preparations described herein have predetermined or target levels of glycans (e.g., branched glycans having a sialic acid on an α1,3 arm, and/or branched glycans having a sialic acid on an α1,6 arm, and/or branched glycans having a sialic acid on an α1,3 arm and on an α1,6 arm), where the predetermined levels are substantially similar to or different from (e.g., higher or lower than) levels of glycans (e.g., branched glycans having a sialic acid on an α1,3 arm, and/or branched glycans having a sialic acid on an α1,6 arm, and/or branched glycans having a sialic acid on an α1,3 arm and on an α1,6 arm) in a reference polypeptide product (e.g., glycoprotein product). Nonlimiting, exemplary reference glycoprotein products can include abatacept (Orencia®, Bristol-Myers Squibb), abciximab (ReoPro®, Roche), adalimumab (Humira®, Bristol-Myers Squibb), aflibercept (Eylea®, Regeneron Pharmaceuticals), alefacept (Amevive®, Astellas Pharma), alemtuzumab (Campath®, Genzyme/Bayer), basiliximab (Simulect®, Novartis), belatacept (Nulojix®, Bristol-Myers Squibb), belimumab (Benlysta®, GlaxoSmithKline), bevacizumab (Avastin®, Roche), canakinumab (Maris®, Novartis), brentuximab vedotin (Adcetris®, Seattle Genetics), certolizumab (CIMZIA®, UCB, Brussels, Belgium), cetuximab (Erbitux®, Merck-Serono), daclizumab (Zenapax®, Hoffmann-La Roche), denileukin diftitox (Ontak®, Eisai), denosumab (Prolia®, Amgen; Xgeva®, Amgen), eculizumab (Soliris®, Alexion Pharmaceuticals), efalizumab (Raptiva®, Genentech), etanercept (Enbrel®, Amgen-Pfizer), gemtuzumab (Mylotarg®, Pfizer), golimumab (Simponi®, Janssen), ibritumomab (Zevalin®, Spectrum Pharmaceuticals), infliximab (Remicade®, Centocor), ipilimumab (Yervoy™, Bristol-Myers Squibb), muromonab (Orthoclone OKT3®, Janssen-Cilag), natalizumab (Tysabri®, Biogen Idec, Elan), ofatumumab (Arzerra®, GlaxoSmithKline), omalizumab (Xolair®, Novartis), palivizumab (Synagis®, Medlmmune), panitumumab (Vectibix®, Amgen), ranibizumab (Lucentis®, Genentech), rilonacept (Arcalyst®, Regeneron Pharmaceuticals), rituximab (MabThera®, Roche), tocilizumab (Actemra®, Genentech; RoActemra, Hoffman-La Roche) tositumomab (Bexxar®, GlaxoSmithKline), and trastuzumab (Herceptin®, Roche).

In some parts of the disclosure, a level of one or more glycans (e.g., branched glycans having a sialic acid on an α1,3 arm, and/or branched glycans having a sialic acid on an α1,6 arm, and/or branched glycans having a sialic acid on an α1,3 arm and on an α1,6 arm) in a reference polypeptide product is determined by analyzing one or more preparations (e.g., one or more lots) of the reference polypeptide. In some parts of the disclosure, a level of one or more glycans (e.g., branched glycans having a sialic acid on an α1,3 arm, and/or branched glycans having a sialic acid on an α1,6 arm, and/or branched glycans having a sialic acid on an α1,3 arm and on an α1,6 arm) in a reference polypeptide product is a range of the one or more glycans in two or more preparations of the reference polypeptide (e.g., two or more lots of the reference polypeptide product). In some parts of the disclosure, a level of one or more glycans is a range (e.g., spanning a lowest level of the one or more glycans to a highest level of the one or more glycans) in two or more lots of the reference polypeptide product.

### N-Linked Glycosylation

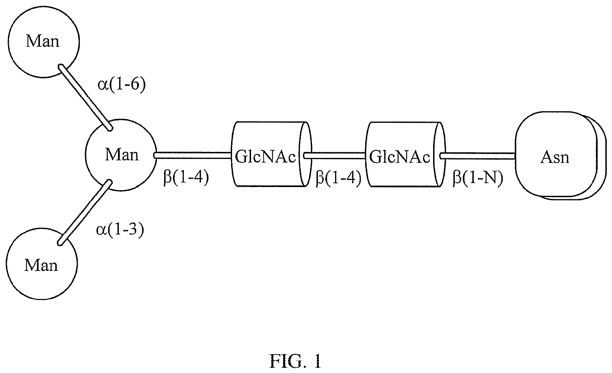

N-linked oligosaccharide chains are added to a protein in the lumen of the endoplasmic reticulum (see Molecular Biology of the Cell, Garland Publishing, Inc. (Alberts et al., 1994)). Specifically, an initial oligosaccharide (typically 14-sugar) is added to the amino group on the side chain of an asparagine residue contained within the target consensus sequence of Asn-X-Ser/Thr, where X may be any amino acid except proline. The structure of this initial oligosaccharide is common to most eukaryotes, and contains 3 glucose, 9 mannose, and 2 N-acetylglucosamine residues. This initial oligosaccharide chain can be trimmed by specific glycosidase enzymes in the endoplasmic reticulum, resulting in a short, branched core oligosaccharide composed of two N-acetylglucosamine and three mannose residues (depicted in Figure 1, linked to an asparagine residue). One of the branches is referred to in the art as the "α1,3 arm", and the second branch is referred to as the "α1,6 arm", as denoted in Figure 1.

N-glycans can be subdivided into three distinct groups called "high mannose type", "hybrid type", and "complex type", with a common pentasaccharide core (Man (alpha1,6)-(Man(alpha1,3))-Man(beta1,4)-GlcpNAc(beta 1,4)-GlcpNAc(beta 1,N)-Asn) occurring in all three groups.

After initial processing in the endoplasmic reticulum, the glycoprotein is transported to the Golgi where further processing may take place. If the glycan is transferred to the Golgi before it is completely trimmed to the core pentasaccharide structure, it results in a "high-mannose glycan".

Additionally or alternatively, one or more monosaccharides units of N-acetylglucosamine may be added to core mannose subunits to form a "complex glycan". Galactose may be added to N-acetylglucosamine subunits, and sialic acid subunits may be added to galactose subunits, resulting in chains that terminate with any of a sialic acid, a galactose or an N-acetylglucosamine residue. Additionally, a fucose residue may be added to an N-acetylglucosamine residue of the core oligosaccharide. Each of these additions is catalyzed by specific glycosyl transferases, known in the art.

Sialic acids are a family of 9-carbon monosaccharides with heterocyclic ring structures. They bear a negative charge via a carboxylic acid group attached to the ring as well as other chemical decorations including N-acetyl and N-glycolyl groups. The two main types of sialyl residues found in glycoproteins produced in mammalian expression systems are N-acetyl-neuraminic acid (NeuAc) and N-glycolylneuraminic acid (NeuGc). These usually occur as terminal structures attached to galactose (Gal) residues at the non-reducing termini of both N- and O-linked glycans. The glycosidic linkage configurations for these sialyl groups can be either α2,3 or α2,6.

"Hybrid glycans" comprise characteristics of both high-mannose and complex glycans. For example, one branch of a hybrid glycan may comprise primarily or exclusively mannose residues, while another branch may comprise N-acetylglucosamine, sialic acid, and/or galactose sugars.

### N-linked Glycosylation in Antibodies

Antibodies are glycosylated at conserved, N-linked glycosylation sites in the Fc regions of immunoglobulin heavy chains. For example, each heavy chain of an IgG antibody has a single N-linked glycosylation site at Asn297 of the CH2 domain (see Jefferis, Nature Reviews 8:226-234 (2009)). IgA antibodies have N-linked glycosylation sites within the CH2 and CH3 domains, IgE antibodies have N-linked glycosylation sites within the CH3 domain, and IgM antibodies have N-linked glycosylation sites within the CH1, CH2, CH3, and CH4 domains (see Arnold et al., J. Biol. Chem. 280:29080-29087 (2005); Mattu et al., J. Biol. Chem. 273:2260-2272 (1998); Nettleton et al., Int. Arch. Allergy Immunol. 107:328-329 (1995)).

Each antibody isotype has a distinct variety of N-linked carbohydrate structures in the constant regions. For example, IgG has a single N-linked biantennary carbohydrate at Asn297 of the CH2 domain in each Fc polypeptide of the Fc region, which also contains the binding sites for C1q and FcyR (see Jefferis et al., Immunol. Rev. 163:59-76 (1998); and Wright et al., Trends Biotech 15:26-32 (1997)). For human IgG, the core oligosaccharide normally consists of GlcNAc₂Man₃GlcNAc, with differing numbers of outer residues. Variation among individual IgG can occur via attachment of galactose and/or galactose-sialic acid at one or both terminal GlcNAc or via attachment of a third GlcNAc arm (bisecting GlcNAc), and/or attachment of fucose.

### Antibodies

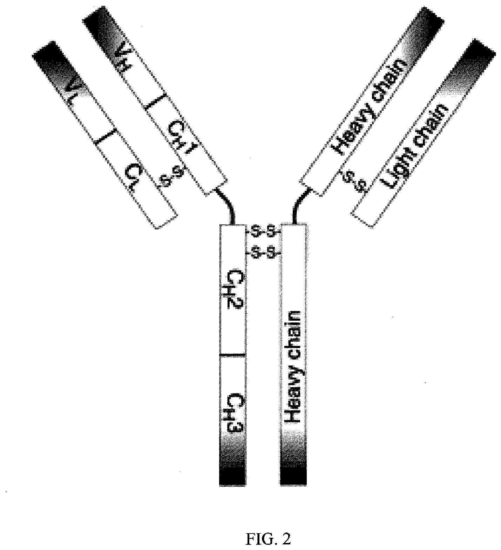

The basic structure of an IgG antibody is illustrated in Figure 2. As shown in Figure 2, an IgG antibody consists of two identical light polypeptide chains and two identical heavy polypeptide chains linked together by disulphide bonds. The first domain located at the amino terminus of each chain is variable in amino acid sequence, providing antibody binding specificities found in each individual antibody. These are known as variable heavy (VH) and variable light (VL) regions. The other domains of each chain are relatively invariant in amino acid sequence and are known as constant heavy (CH) and constant light (CL) regions. As shown in Figure 2, for an IgG antibody, the light chain includes one variable region (VL) and one constant region (CL). An IgG heavy chain includes a variable region (VH), a first constant region (CH1), a hinge region, a second constant region (CH2), and a third constant region (CH3). In IgE and IgM antibodies, the heavy chain includes an additional constant region (CH4).

Antibodies described herein can include, for example, monoclonal antibodies, polyclonal antibodies (e.g., IVIG), multispecific antibodies, human antibodies, humanized antibodies, camelized antibodies, chimeric antibodies, single-chain Fvs (scFv), disulfide-linked Fvs (sdFv), and anti-idiotypic (anti-Id) antibodies, and antigen-binding fragments of any of the above. Antibodies can be of any type (e.g., IgG, IgE, IgM, IgD, IgA and IgY), class (e.g., IgG1, IgG2, IgG3, IgG4, IgA1 and IgA2) or subclass.

The term "Fc fragment", as used herein, refers to one or more fragments of an Fc region that retains an Fc function and/or activity described herein, such as binding to an Fc receptor. Examples of such fragments include fragments that include an N-linked glycosylation site of an Fc region (e.g., an Asn297 of an IgG heavy chain or homologous sites of other antibody isotypes), such as a CH2 domain. The term "antigen binding fragment" of an antibody, as used herein, refers to one or more fragments of an antibody that retain the ability to specifically bind to an antigen. Examples of binding fragments encompassed within the term "antigen binding fragment" of an antibody include a Fab fragment, a F(ab')₂ fragment, a Fd fragment, a Fv fragment, a scFv fragment, a dAb fragment (Ward et al., (1989) Nature 341:544-546), and an isolated complementarity determining region (CDR). These antibody fragments can be obtained using conventional techniques known to those with skill in the art, and fragments can be screened for utility in the same manner as are intact antibodies.

Glycoproteins (e.g., antibodies), or fragments thereof, for use as substrates for an ST6 sialyltransferase described herein, can be produced by any method known in the art for synthesizing glycoproteins (e.g., antibodies) (see, e.g., Harlow et al., Antibodies: A Laboratory Manual, (Cold Spring Harbor Laboratory Press, 2nd ed. 1988); Brinkman et al., 1995, J. Immunol. Methods 182:41-50; WO 92/22324; WO 98/46645). Chimeric antibodies can be produced using methods described in, e.g., Morrison, 1985, Science 229:1202, and humanized antibodies by methods described in, e.g., U.S. Pat. No. 6,180,370.

Additional reference antibodies described herein are bispecific antibodies and multivalent antibodies, as described in, e.g., Segal et al., J. Immunol. Methods 248:1-6 (2001); and Tutt et al., J. Immunol. 147: 60 (1991).

### Glycoprotein Conjugates

The disclosure includes glycoproteins (or Fc regions or Fc fragments containing one or more N-glycosylation sites thereof) that are conjugated or fused to one or more heterologous moieties. Heterologous moieties include, but are not limited to, peptides, polypeptides, proteins, fusion proteins, nucleic acid molecules, small molecules, mimetic agents, synthetic drugs, inorganic molecules, and organic molecules. In some instances, a glycoprotein conjugate is a fusion protein that comprises a peptide, polypeptide, protein scaffold, scFv, dsFv, diabody, Tandab, or an antibody mimetic fused to an Fc region, such as a glycosylated Fc region. A fusion protein can include a linker region connecting an Fc region to a heterologous moiety (see, e.g., Hallewell et al. (1989), J. Biol. Chem. 264, 5260-5268; Alfthan et al. (1995), Protein Eng. 8, 725-731; Robinson & Sauer (1996)).

Exemplary, nonlimiting reference glycoprotein conjugate products include abatacept (Orencia®, Bristol-Myers Squibb), aflibercept (Eylea®, Regeneron Pharmaceuticals), alefacept (Amevive®, Astellas Pharma), belatacept (Nulojix®, Bristol-Myers Squibb), denileukin diftitox (Ontak®, Eisai), etanercept (Enbrel®, Amgen-Pfizer), and rilonacept (Arcalyst®, Regeneron Pharmaceuticals).

In some instances, a glycoprotein conjugate includes an Fc region (or an Fc fragment containing one or more N-glycosylation sites thereof) conjugated to a heterologous polypeptide of at least 10, at least 20, at least 30, at least 40, at least 50, at least 60, at least 70, at least 80, at least 90 or at least 100 amino acids.

In some instances, a glycoprotein conjugate includes an Fc region (or an Fc fragment containing one or more N-glycosylation sites thereof) conjugated to one or more marker sequences, such as a peptide to facilitate purification. A particular marker amino acid sequence is a hexa-histidine peptide, such as the tag provided in a pQE vector (QIAGEN, Inc., 9259 Eton Avenue, Chatsworth, Calif., 91311). Other peptide tags useful for purification include, but are not limited to, the hemagglutinin "HA" tag, which corresponds to an epitope derived from the influenza hemagglutinin protein (Wilson et al., 1984, Cell 37:767) and the "Flag" tag.

In other instances, a glycoprotein conjugate includes an Fc region (or Fc fragment containing one or more N-glycosylation sites thereof) conjugated to a diagnostic or detectable agent. Such fusion proteins can be useful for monitoring or prognosing development or progression of disease or disorder as part of a clinical testing procedure, such as determining efficacy of a particular therapy. Such diagnosis and detection can be accomplished by coupling a glycoprotein to detectable substances including, but not limited to, various enzymes, such as but not limited to horseradish peroxidase, alkaline phosphatase, beta-galactosidase, or acetylcholinesterase; prosthetic groups, such as, but not limited to, streptavidin/biotin and avidin/biotin; fluorescent materials, such as, but not limited to, umbelliferone, fluorescein, fluorescein isothiocynate, rhodamine, dichlorotriazinylamine fluorescein, dansyl chloride or phycoerythrin; luminescent materials, such as, but not limited to, luminol; bioluminescent materials, such as but not limited to, luciferase, luciferin, and aequorin; radioactive materials, such as but not limited to iodine (¹³¹I, ¹²⁵I, ¹²³I), carbon (¹⁴C), sulfur (³⁵S), tritium (³H), indium (¹¹⁵In, ¹¹³In, ¹¹²In, ¹¹¹In), technetium (⁹⁹Tc), thallium (²⁰¹Ti), gallium (⁶⁸Ga, ⁶⁷Ga), palladium (¹⁰³Pd), molybdenum (⁹⁹Mo), xenon (¹³³Xe), fluorine (¹⁸F), ¹⁵³Sm, ¹⁷⁷Lu, ¹⁵³Gd, ¹⁵⁹Gd, ¹⁴⁹Pm, ¹⁴⁰La, ¹⁶⁹Yb, ¹⁷⁵Yb, ¹⁶⁶Ho, ⁹⁰Y, ⁴⁷Sc, ¹⁸⁶Re, ¹⁸⁸Re, ¹⁴²Pr, ¹⁰⁵Rh, ⁹⁷Ru, ⁶⁸Ge, ⁵⁷Co, ⁶⁵Zn, ⁸⁵Sr, ³²P, ⁵¹Cr, ⁵⁴Mn, ⁷⁵Se, ¹¹³Sn, and ¹¹⁷Sn; positron emitting metals using various positron emission tomographies, non-radioactive paramagnetic metal ions, and molecules that are radiolabelled or conjugated to specific radioisotopes.

Techniques for conjugating therapeutic moieties to antibodies are well known (see, e.g., Arnon et al., "Monoclonal Antibodies For Immunotargeting Of Drugs In Cancer Therapy", in Monoclonal Antibodies And Cancer Therapy, Reisfeld et al. (eds.), pp. 243-56. (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987)).

### Sialyltransferase Polypeptides

Methods and compositions described herein include the use of a sialyltransferase enzyme, e.g., an α2,6 sialyltransferase (e.g., ST6 Gal-I). A number of ST6 sialyltransferases are known in the art and are commercially available (see, e.g., Takashima, Biosci. Biotechnol. Biochem. 72:1155-1167 (2008); Weinstein et al., J. Biol. Chem. 262:17735-17743 (1987)). ST6 Gal-I catalyzes the transfer of sialic acid from a sialic acid donor (e.g., cytidine 5'-monophospho-N-acetyl neuraminic acid) to a terminal galactose residue of glycans through an α2,6 linkage. The sialic acid donor reaction product is cytidine 5'-monophosphate. In some embodiments, an ST6 sialyltransferase has or includes an amino acid sequence set forth in SEQ ID NO:2, SEQ ID NO:3, or in amino acid residues 95-416 of SEQ ID NO:1, or a characteristic sequence element thereof or therein. In some parts of the disclosure, an ST6 sialyltransferase has at least 100%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, or 70% overall sequence identity with one or more of SEQ ID NO:2, SEQ ID NO:3, or amino acid residues 95-416 of SEQ ID NO:1. Alternatively or additionally, in some parts of the disclosure, an ST6 sialyltransferase includes at least about 5, 10, 15, 20, 25, 30, 35, 40, 45, 50, 75, 100, or 150 or more contiguous amino acid residues found in SEQ ID NO:2, SEQ ID NO:3, or amino acid residues 95-416 of SEQ ID NO:1.

In some parts of the disclosure, an ST6 sialyltransferase differs from an amino acid sequence as set forth in SEQ ID NO:2, SEQ ID NO:3, or in amino acid residues 95-416 of SEQ ID NO:1, or characteristic sequence elements thereof or therein, by one or more amino acid residues. For example, in some parts of the disclosure, the difference is a conservative or nonconservative substitution of one or more amino acid residues. Conservative substitutions are those that substitute a given amino acid in a polypeptide by another amino acid of similar characteristics. Typical conservative substitutions are the following replacements: replacement of an aliphatic amino acid, such as alanine, valine, leucine, and isoleucine, with another aliphatic amino acid; replacement of a serine with a threonine or vice versa; replacement of an acidic residue, such as aspartic acid and glutamic acid, with another acidic residue; replacement of a residue bearing an amide group, such as asparagine and glutamine, with another residue bearing an amide group; exchange of a basic residue, such as lysine and arginine, with another basic residue; and replacement of an aromatic residue, such as phenylalanine and tyrosine, with another aromatic residue.

In some parts of the disclosure, an ST6 sialyltransferase polypeptide includes a substituent group on one or more amino acid residues. Still other useful polypeptides are associated with (e.g., fused, linked, or coupled to) another moiety (e.g., a peptide or molecule). For example, an ST6 sialyltransferase polypeptides can be fused, linked, or coupled to an amino acid sequence (e.g., a leader sequence, a secretory sequence, a proprotein sequence, a second polypeptide, or a sequence that facilitates purification, enrichment, or stabilization of the polypeptide).

### Methods of Sialylating Glycoproteins

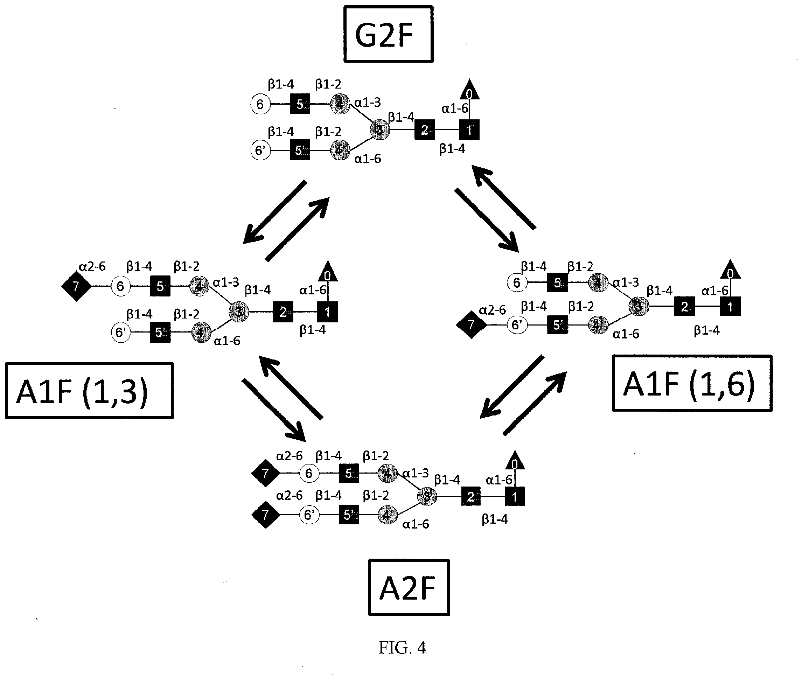

ST6 Gal-I sialyltransferase catalyzes the transfer of sialic acid from a sialic acid donor (e.g., cytidine 5'-monophospho-N-acetyl neuraminic acid) to a terminal galactose residue of glycans through an α2,6 linkage. The present disclosure exploits the discovery that ST6 sialyltransferase catalyzes the transfer of sialic acid to branched glycans (e.g., Fc branched glycans) comprising an α1,3 arm and an α1,6 arm in an ordered fashion. As shown in Figure 4, ST6 sialyltransferase transfers a sialic acid to an α1,3 arm of a branched glycan, which can be followed by transfer of a second sialic acid to an α1,6 arm (yielding a disialylated branched glycan), and can further be followed by removal of sialic acid from an α1,3 arm (yielding a branched glycan having a sialic acid on an α1,6 arm). Accordingly, by controlling and/or modulating activity (e.g., kinetics) of ST6 sialyltransferase, glycoproteins having particular sialylation patterns can be produced.

Any parameter generally known to affect enzyme kinetics can be controlled and/or modulated to produce a glycoprotein preparation having a predetermined or target level of sialic acid on an α1,3 arm of a branched glycan, on an α1,6 arm of a branched glycan, and/or on an α1,3 arm and an α1,6 arm of a branched glycan. For example, reaction time, ST6 sialyltransferase concentration and/or specific activity, branched glycan concentration, sialic acid donor concentration, sialic acid donor reaction product concentration, pH, buffer composition, and/or temperature can be controlled and/or modulated to produce a glycoprotein preparation having a desired level of sialylation (e.g., α1,3 arm and/or α1,6 arm sialylation).

In some parts of the disclosure, to preferentially sialylate an α1,3 arm of branched glycans (e.g., having an α1,3 arm and an α1,6 arm), branched glycans are contacted in vitro with an ST6 sialyltransferase under limited reaction conditions. Such limited reaction conditions are selected such that addition of a sialic acid to an α1,3 arm is enhanced relative to addition of a sialic acid to an α1,6 arm (e.g., rate of transfer of a sialic acid to an α1,3 arm ("Rₐ^{1,3}") exceeds rate of transfer of a sialic acid to an α1,6 arm ("Rₐ^{1,6}"). In some parts of the disclosure, limited reaction conditions are further selected such that removal of a sialic acid from an α1,6 arm is enhanced relative to addition of a sialic acid to an α1,6 arm (e.g., rate of removal of a sialic acid from an α1,6 arm ("Rᵣ^{1,6}") exceeds rate of transfer of a sialic acid to an α1,6 arm ("Rₐ^{1,6}"). Limited reaction conditions can include, for example, reduced reaction time, reduced enzyme concentration and/or activity, reduced amount of branched glycans, reduced level of sialic acid donor, and/or reduced temperature.

In some parts of the disclosure, to preferentially sialylate an α1,6 arm of branched glycans (e.g., having an α1,3 arm and an α1,6 arm), branched glycans can be contacted in vitro with an ST6 sialyltransferase under extended reaction conditions. Such extended reaction conditions are selected such that addition of a sialic acid to an α1,6 arm is enhanced relative to removal of a sialic acid from an α1,6 arm (e.g., rate of transfer of a sialic acid to an α1,6 arm ("Rₐ^{1,6}") exceeds rate of removal of a sialic acid from an α1,6 arm ("Rᵣ^{1,6}")). In some embodiments, extended reaction conditions are further selected such that, after initial conditions that enhance addition of sialic acid to an α1,3 arm, conditions are extended such that removal of a sialic acid from an α1,3 arm is eventually enhanced relative to addition of a sialic acid to an α1,3 arm (e.g., rate of removal of a sialic acid from an α1,3 arm ("Rᵣ^{1,3}") exceeds rate of transfer of a sialic acid to an α1,3 arm ("Rₐ^{1,3}")). Extended reaction conditions can include, for example, increased reaction time, increased enzyme concentration and/or activity, increased amount of branched glycans, increased level of sialic acid donor, and/or increased temperature.

In some parts of the disclosure, to preferentially sialylate both an α1,3 arm and an α1,6 arm of branched glycans (e.g., having an α1,3 arm and an α1,6 arm), branched glycans are contacted in vitro with an ST6 sialyltransferase under intermediate reaction conditions. Such intermediate reaction conditions are selected such that addition of a sialic acid to an α1,3 arm is enhanced relative to removal of a sialic acid from an α1,3 arm (e.g., rate of transfer of a sialic acid to an α1,3 arm ("Rₐ^{1,3}") exceeds rate of removal of a sialic acid from an α1,3 arm ("Rᵣ^{1,3}"). In some parts of the disclosure, intermediate reaction conditions are further selected such that addition of a sialic acid to an α1,6 arm is enhanced relative to removal of a sialic acid from an α1,6 arm (e.g., rate of addition of a sialic acid to an α1,6 arm ("Rₐ^{1,6}") exceeds rate of removal of a sialic acid from an α1,6 arm ("Rᵣ^{1,6}"). Intermediate reaction conditions can include, for example, intermediate reaction time, intermediate enzyme concentration and/or activity, intermediate amount of branched glycans, intermediate level of sialic acid donor, and/or intermediate temperature. In some parts of the disclosure, intermediate reaction conditions further include supplementing the sialic acid donor at least once during the reaction. In some parts of the disclosure, intermediate reaction conditions further include removing a sialic acid donor reaction product at least once during the reaction. In some parts of the disclosure, intermediate reaction conditions further include supplementing the sialic acid donor reaction product at least once during the reaction.

In some parts of the disclosure, a glycoprotein, e.g., a glycosylated antibody, is sialylated after the glycoprotein is produced. For example, a glycoprotein can be recombinantly expressed in a host cell (as described herein) and purified using standard methods. The purified glycoprotein is then contacted with an ST6 sialyltransferase (e.g., a recombinantly expressed and purified ST6 sialyltransferase) in the presence of reaction conditions as described herein. In certain parts of the disclosure, the conditions include contacting the purified glycoprotein with an ST6 sialyltransferase in the presence of a sialic acid donor, e.g., cytidine 5'-monophospho-N-acetyl neuraminic acid, manganese, and/or other divalent metal ions. In some embodiments, IVIG is used in a sialylation method described herein.

In some parts of the disclosure, chemoenzymatic sialylation is used to sialylate glycoproteins. Briefly, this method involves sialylation of a purified branched glycan, followed by incorporation of the sialylated branched glycan en bloc onto a polypeptide to produce a sialylated glycoprotein.

A branched glycan can be synthesized de novo using standard techniques or can be obtained from a glycoprotein preparation (e.g., a recombinant glycoprotein, Fc, or IVIG) using an appropriate enzyme, such as an endoglycosidase (e.g., EndoH or EndoF). After sialylation of the branched glycan, the sialylated branched glycan can be conjugated to a polypeptide using an appropriate enzyme, such as a transglycosidase, to produce a sialylated glycoprotein.

In one exemplary method, a purified branched N-glycan is obtained from a glycoprotein (e.g., a glycoprotein preparation, e.g., IVIG) using an endoglycosidase. The purified branched N-glycan is then chemically activated on the reducing end to form a chemically active intermediate. The branched N-glycan is then further processed, trimmed, and/or glycosylated using appropriate known glycosidases. The branched glycan is then sialylated using an ST6 sialylation as described herein. After engineering, the desired branched N-glycan is transferred onto a glycoprotein using a transglycosidase (such as a transglycosidase in which glycosidic activity has been attenuated using genetically engineering).

In some parts of the disclosure, a branched glycan used in methods described herein is a galactosylated branched glycan (e.g., includes a terminal galactose residue). In some parts of the disclosure, a branched glycan is galactosylated before being sialylated using a method described herein. In some parts of the disclosure, a branched glycan is first contacted with a galactosyltransferase (e.g., a beta-1,3-galactosyltransferase) and subsequently contacted with an ST6 sialyltransferase as described herein. In some parts of the disclosure, a galactosylated glycan is purified before being contacted with an ST6 sialyltransferase. In some parts of the disclosure, a galactosylated glycan is not purified before being contacted with an ST6 sialyltransferase. In some parts of the disclosure, a branched glycan is contacted with a galactosyltransferase and an ST6 sialyltransferase in a single step.

In some parts of the disclosure, a host cell is genetically engineered to express a glycoprotein described herein and one or more sialyltransferase enzymes, e.g., an ST6 sialyltransferase. In some parts of the disclosure, the host cell is genetically engineered to further express a galactosyltransferase. The genetically engineered host cell can be cultured under conditions sufficient to produce a particular sialylated glycoprotein. For example, to produce glycoproteins preferentially sialylated on α1,3 arms of branched glycans, a host cell can be genetically engineered to express a relatively low level of ST6 sialyltransferase, whereas to produce glycoproteins preferentially sialylated on α1,6 arms of branched glycans, a host cell can be genetically engineered to express a relatively high level of ST6 sialyltransferase. In some parts of the disclosure, to produce glycoproteins preferentially sialylated on α1,3 arms of branched glycans, a genetically engineered host cell can be cultured in a relatively low level of sialic acid donor, whereas to produce glycoproteins preferentially sialylated on α1,6 arms of branched glycans, a genetically engineered host cell can be cultured in a relatively high level of sialic acid donor.

### Recombinant Gene Expression

In accordance with the present disclosure, there may be employed conventional molecular biology, microbiology, and recombinant DNA techniques within the skill of the art. Such techniques are described in the literature (see, e.g., Green & Sambrook, Molecular Cloning: A Laboratory Manual, Fourth Edition (2012) Cold Spring Harbor Laboratory Press, Cold Spring Harbor, N.Y.; DNA Cloning: A Practical Approach, Volumes I-IV (D. N. Glover ed. 1995; 1996); Oligonucleotide Synthesis (M. J. Gait ed. 1984); Nucleic Acid Hybridisation (B. D. Hames & S. J. Higgins eds. (1985)); Transcription And Translation (B. D. Hames & S. J. Higgins, eds. (1984)); Culture of Animal Cells, Sixth Edition (R. I. Freshney, ed. (2010)); Immobilized Cells and Enzymes (IRL Press, (1986)); B. Perbal, A Practical Guide To Molecular Cloning, Second Edition (1988); F. M. Ausubel et al. (eds.), Current Protocols in Molecular Biology, John Wiley & Sons, Inc. (1995).

Recombinant expression of a gene, such as a gene encoding a polypeptide, such as an antibody or a sialyltransferase described herein, can include construction of an expression vector containing a polynucleotide that encodes the polypeptide. Once a polynucleotide has been obtained, a vector for the production of the polypeptide can be produced by recombinant DNA technology using techniques known in the art. Known methods can be used to construct expression vectors containing polypeptide coding sequences and appropriate transcriptional and translational control signals. These methods include, for example, in vitro recombinant DNA techniques, synthetic techniques, and in vivo genetic recombination.

An expression vector can be transferred to a host cell by conventional techniques, and the transfected cells can then be cultured by conventional techniques to produce polypeptide.

A variety of host expression vector systems can be used (see, e.g., U.S. Pat. No. 5,807,715). Such host-expression systems can be used to produce polypeptides and, where desired, subsequently purified. Such host expression systems include microorganisms such as bacteria (e.g., E. coli and B. subtilis) transformed with recombinant bacteriophage DNA, plasmid DNA or cosmid DNA expression vectors containing polypeptide coding sequences; yeast (e.g., Saccharomyces and Pichia) transformed with recombinant yeast expression vectors containing polypeptide coding sequences; insect cell systems infected with recombinant virus expression vectors (e.g., baculovirus) containing polypeptide coding sequences; plant cell systems infected with recombinant virus expression vectors (e.g., cauliflower mosaic virus, CaMV; tobacco mosaic virus, TMV) or transformed with recombinant plasmid expression vectors (e.g. Ti plasmid) containing polypeptide coding sequences; or mammalian cell systems (e.g., COS, CHO, BHK, 293, NSO, and 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter).

For bacterial systems, a number of expression vectors can be used, including, but not limited to, the E. coli expression vector pUR278 (Ruther et al., 1983, EMBO 12:1791); pIN vectors (Inouye & Inouye, 1985, Nucleic Acids Res. 13:3101-3109; Van Heeke & Schuster, 1989, J. Biol. Chem. 24:5503-5509); and the like. pGEX vectors can also be used to express foreign polypeptides as fusion proteins with glutathione 5-transferase (GST).

For expression in mammalian host cells, viral-based expression systems can be utilized (see, e.g., Logan & Shenk, 1984, Proc. Natl. Acad. Sci. USA 8 1:355-359). The efficiency of expression can be enhanced by the inclusion of appropriate transcription enhancer elements, transcription terminators, etc. (see, e.g., Bittner et al., 1987, Methods in Enzymol. 153:516-544).

In addition, a host cell strain can be chosen that modulates the expression of the inserted sequences, or modifies and processes the gene product in the specific fashion desired. Different host cells have characteristic and specific mechanisms for the post-translational processing and modification of proteins and gene products. Appropriate cell lines or host systems can be chosen to ensure the correct modification and processing of the polypeptide expressed. Such cells include, for example, established mammalian cell lines and insect cell lines, animal cells, fungal cells, and yeast cells. Mammalian host cells include, but are not limited to, CHO, VERY, BHK, HeLa, COS, MDCK, 293, 3T3, W138, BT483, Hs578T, HTB2, BT20 and T47D, NSO (a murine myeloma cell line that does not endogenously produce any immunoglobulin chains), CRL7O3O and HsS78Bst cells.

For long-term, high-yield production of recombinant proteins, host cells are engineered to stably express a polypeptide. Host cells can be transformed with DNA controlled by appropriate expression control elements known in the art, including promoter, enhancer, sequences, transcription terminators, polyadenylation sites, and selectable markers. Methods commonly known in the art of recombinant DNA technology can be used to select a desired recombinant clone.

Once a glycoprotein described herein been produced by recombinant expression, it may be purified by any method known in the art for purification, for example, by chromatography (e.g., ion exchange, affinity, and sizing column chromatography), centrifugation, differential solubility, or by any other standard technique for the purification of proteins. For example, an antibody can be isolated and purified by appropriately selecting and combining affinity columns such as Protein A column with chromatography columns, filtration, ultra filtration, salting-out and dialysis procedures (see Antibodies: A Laboratory Manual, Ed Harlow, David Lane, Cold Spring Harbor Laboratory, 1988). Further, as described herein, a glycoprotein can be fused to heterologous polypeptide sequences to facilitate purification. Glycoproteins having desired sugar chains can be separated with a lectin column by methods known in the art (see, e.g., WO 02/30954).

### Glycan Evaluation

Glycans of glycoproteins can be evaluated using any methods known in the art. For example, sialylation of glycan compositions (e.g., level of branched glycans that are sialylated on an α1,3 arm and/or an α1,6 arm) can be characterized using methods described in, e.g., Barb, Biochemistry 48:9705-9707 (2009); Anumula, J. Immunol. Methods 382:167-176 (2012); Gilar et al., Analytical Biochem. 417:80-88 (2011); Wuhrer et al., J. Chromatogr. B. 849:115-128 (2007). In some embodiments, in addition to evaluation of sialylation of glycans, one or more parameters described in Table 1 are evaluated.

In some instances, glycan structure and composition as described herein are analyzed, for example, by one or more, enzymatic, chromatographic, mass spectrometry (MS), chromatographic followed by MS, electrophoretic methods, electrophoretic methods followed by MS, nuclear magnetic resonance (NMR) methods, and combinations thereof. Exemplary enzymatic methods include contacting a glycoprotein preparation with one or more enzymes under conditions and for a time sufficient to release one or more glycan(s) (e.g., one or more exposed glycan(s)). In some instances, the one or more enzymes include(s) PNGase F. Exemplary chromatographic methods include, but are not limited to, Strong Anion Exchange chromatography using Pulsed Amperometric Detection (SAX-PAD), liquid chromatography (LC), high performance liquid chromatography (HPLC), ultra performance liquid chromatography (UPLC), thin layer chromatography (TLC), amide column chromatography, and combinations thereof. Exemplary mass spectrometry (MS) include, but are not limited to, tandem MS, LC-MS, LC-MS/MS, matrix assisted laser desorption ionisation mass spectrometry (MALDI-MS), Fourier transform mass spectrometry (FTMS), ion mobility separation with mass spectrometry (IMS-MS), electron transfer dissociation (ETD-MS), and combinations thereof. Exemplary electrophoretic methods include, but are not limited to, capillary electrophoresis (CE), CE-MS, gel electrophoresis, agarose gel electrophoresis, acrylamide gel electrophoresis, SDS-polyacrylamide gel electrophoresis (SDS-PAGE) followed by Western blotting using antibodies that recognize specific glycan structures, and combinations thereof. Exemplary nuclear magnetic resonance (NMR) include, but are not limited to, one-dimensional NMR (1D-NMR), two-dimensional NMR (2D-NMR), correlation spectroscopy magnetic-angle spinning NMR (COSY-NMR), total correlated spectroscopy NMR (TOCSY-NMR), heteronuclear single-quantum coherence NMR (HSQC-NMR), heteronuclear multiple quantum coherence (HMQC-NMR), rotational nuclear overhauser effect spectroscopy NMR (ROESY-NMR), nuclear overhauser effect spectroscopy (NOESY-NMR), and combinations thereof.

In some instances, techniques described herein may be combined with one or more other technologies for the detection, analysis, and or isolation of glycans or glycoproteins. For example, in certain instances, glycans are analyzed in accordance with the present disclosure using one or more available methods (to give but a few examples, see Anumula, Anal. Biochem., 350(1):1, 2006; Klein et al., Anal. Biochem., 179:162, 1989; and/or Townsend, R.R. Carbohydrate Analysis" High Performance Liquid Chromatography and Capillary Electrophoresis., Ed. Z. EI Rassi, pp 181-209, 1995; WO2008/128216; WO2008/128220; WO2008/128218; WO2008/130926; WO2008/128225; WO2008/130924; WO2008/128221; WO2008/128228; WO2008/128227; WO2008/128230; WO2008/128219; WO2008/128222; WO2010/071817; WO2010/071824; WO2010/085251; WO2011/069056; and WO2011/127322. For example, in some instances, glycans are characterized using one or more of chromatographic methods, electrophoretic methods, nuclear magnetic resonance methods, and combinations thereof. In some instances, methods for evaluating one or more target protein specific parameters, e.g., in a glycoprotein preparation, e.g., one or more of the parameters disclosed herein, can be performed by one or more of following methods.

In some instances, methods for evaluating one or more target protein specific parameters, e.g., in a glycoprotein preparation, e.g., one or more of the parameters disclosed herein, can be performed by one or more of following methods.

**Table 1: Exemplary methods of evaluating parameters:**

| **Method(s)** | **Relevant literature** | **Parameter** |
|---|---|---|
| C18 UPLC Mass Spec.* | Chen and Flynn, Anal. Biochem., 370:147-161 (2007) | Glycan(s) (e.g., N-linked glycan, exposed N-linked glycan, glycan detection, glycan identification, and characterization; site specific glycation; glycoform detection (e.g., parameters 1-7); percent glycosylation; and/or aglycosyl) |
| | Chen and Flynn, J. Am. Soc. Mass | |
| | Spectrom., 20:1821-1833 (2009) | |
| Peptide LC-MS (reducing/non-reducing) | Dick et al., Biotechnol. Bioeng., 100:1132-1143 (2008) | C-terminal lysine |
| | Yan et al., J. Chrom. A., 1164:153-161 (2007) | |
| | Chelius et al., Anal. Chem., 78:2370-2376 (2006) | |
| | Miller et al., J. Pharm. Sci., 100:2543-2550 (2011) | |
| LC-MS (reducing/nonreducing/alkylated) | Dick et al., Biotechnol. Bioeng., 100:1132-1143 (2008) | |
| | Goetze et al., Glycobiol., 21:949-959 (2011) | |
| Weak cation exchange (WCX) chromatography | Dick et al., Biotechnol. Bioeng., 100:1132-1143 (2008) | |
| LC-MS (reducing/nonreducing/alkylated) | Dick et al., Biotechnol. Bioeng., 100:1132-1143 (2008) | N-terminal pyroglu |
| | Goetze et al., Glycobiol., 21:949-959 (2011) | |
| PeptideLC-MS (reducing/non-reducing) | Yan et al., J. Chrom. A., 1164:153-161 (2007) | |
| | Chelius et al., Anal. Chem., 78:2370-2376 (2006) | |
| | Miller et al., J. Pharm. Sci., 100:2543-2550 (2011) | |
| Peptide LC-MS (reducing/non-reducing) | Yan et al., J. Chrom. A., 1164:153-161 (2007); | Methionine oxidation |
| | Xie et al., mAbs, 2:379-394 (2010) | |
| Peptide LC-MS (reducing/non-reducing) | Miller et al., J. Pharm. Sci., 100:2543-2550 (2011) | Site specific glycation |
| Peptide LC-MS (reducing/non-reducing) | Wang et al., Anal. Chem., 83:3133-3140 (2011); | Free cysteine |
| | Chumsae et al., Anal. Chem., 81:6449-6457 (2009) | |
| Bioanalyzer (reducing/non-reducing)* | Forrer et al., Anal. Biochem., 334:81-88 (2004) | Glycan (e.g., N-linked glycan, exposed N-linked glycan) (including, for example, glycan detection, identification, and characterization; site specific glycation; glycoform detection; percent glycosylation; and/or aglycosyl) |
| LC-MS (reducing/nonreducing/alkylated)* | Dick et al., Biotechnol. Bioeng., 100:1132-1143 (2008) | Glycan (e.g., N-linked glycan, exposed N-linked glycan) (including, for example, glycan detection, identification, and characterization; site specific glycation; glycoform detection; percent glycosylation; and/or aglycosyl) |
| * Methods include removal (e.g., enzymatic, chemical, and physical) of glycans | Goetze et al., Glycobiol., 21:949-959 (2011) | |
| | Xie et al., mAbs, 2:379-394 (2010) | |
| Bioanalyzer (reducing/non-reducing) | Forrer et al., Anal. Biochem., 334:81-88 (2004) | Light chain : Heavy chain |
| Peptide LC-MS (reducing/non-reducing) | Yan et al., J. Chrom. A., 1164:153-161 (2007) | Non-glycosylation-related peptide modifications (including, for example, sequence analysis and identification of sequence variants; oxidation; succinimide; aspartic acid; and/or site-specific aspartic acid) |
| | Chelius et al., Anal. Chem., 78:2370-2376 (2006) | |
| | Miller et al., J. Pharm. Sci., 100:2543-2550 (2011) | |
| Weak cation exchange (WCX) chromatography | Dick et al., Biotechnol. Bioeng., 100:1132-1143 (2008) | Isoforms (including, for example, charge variants (acidic variants and basic variants); and/or deamidated variants) |
| Anion-exchange chromatography | Ahn et al., J. Chrom. B, 878:403-408 (2010) | Sialylated glycan |
| Anion-exchange chromatography | Ahn et al., J. Chrom. B, 878:403-408 (2010) | Sulfated glycan |
| 1,2-diamino-4,5-methylenedioxybenzene (DMB) labeling method | Hokke et al., FEBS Lett., 275:9-14 (1990) | Sialic acid |
| LC-MS | Johnson et al., Anal. Biochem., 360:75-83 (2007) | C-terminal amidation |
| LC-MS | Johnson et al., Anal. Biochem., 360:75-83 (2007) | N-terminal fragmentation |
| Circular dichroism spectroscopy | Harn et al., Current Trends in Monoclonal Antibody Development and Manufacturing, S. J. Shire et al., eds, 229-246 (2010) | Secondary structure (including, for example, alpha helix content and/or beta sheet content) |
| Intrinsic and/or ANS dye fluorescence | Harn et al., Current Trends in Monoclonal Antibody Development and Manufacturing, S. J. Shire et al., eds, 229-246 (2010) | Tertiary structure (including, for example, extent of protein folding) |
| Hydrogen-deuterium exchange-MS | Houde et al., Anal. Chem., 81:2644-2651 (2009) | Tertiary structure and dynamics (including, for example, accessibility f amide protons to solvent water) |
| Size-exclusion chromatography | Carpenter et al., J. Pharm. Sci., 99:2200-2208 (2010) | Extent of aggregation |
| Analytical ultracentrifugation | Pekar and Sukumar, Anal. Biochem., 367:225-237 (2007) | |

### Glycoprotein Properties

Sialylation patterns of glycoproteins can affect their anti-inflammatory properties. Accordingly, in some embodiments, methods described herein are useful for producing glycoproteins with particular levels of anti-inflammatory properties. In some embodiments, methods described herein are used to produce Fc region-containing glycoproteins containing sialic acid on α1,3 arms of branched glycans with a NeuAc-α2,6-Gal terminal linkages and that exhibit increased anti-inflammatory activity relative to a reference glycoprotein, e.g., a level of anti-inflammatory activity that is at least 10%, at least 20%, at least 30%, at least 40%, at least 50%, at least 60%, at least 70%, at least 80%, at least 90%, at least 100%, at least 125%, at least 150%, at least 175%, at least 200%, at least 250%, at least 300%, or higher, relative to a reference glycoprotein.

In some parts of the disclosure, methods described herein are used to produce Fc region-containing glycoproteins having sialic acids on α1,6 arms or on both α1,3 and α1,6 arms of branched glycans that have the same or alternate properties or biological activities in different disease states.

### Pharmaceutical Compositions and Administration

A glycoprotein of the present disclosure (e.g., an Fc region-containing glycoprotein comprising branched glycans that are sialylated on an α1,3 arm, an α1,6 arm, or both, of the branched glycan in the Fc region, e.g., with a NeuAc-α2,6-Gal terminal linkage), can be incorporated into a pharmaceutical composition. In some parts of the disclosure, such a pharmaceutical composition is useful as an improved composition for the prevention and/or treatment of diseases relative to the corresponding reference glycoprotein. Pharmaceutical compositions comprising a glycoprotein can be formulated by methods known to those skilled in the art. The pharmaceutical composition can be administered parenterally in the form of an injectable formulation comprising a sterile solution or suspension in water or another pharmaceutically acceptable liquid. For example, the pharmaceutical composition can be formulated by suitably combining the sialylated glycoprotein with pharmaceutically acceptable vehicles or media, such as sterile water and physiological saline, vegetable oil, emulsifier, suspension agent, surfactant, stabilizer, flavoring excipient, diluent, vehicle, preservative, binder, followed by mixing in a unit dose form required for generally accepted pharmaceutical practices. The amount of active ingredient included in the pharmaceutical preparations is such that a suitable dose within the designated range is provided.

The sterile composition for injection can be formulated in accordance with conventional pharmaceutical practices using distilled water for injection as a vehicle. For example, physiological saline or an isotonic solution containing glucose and other supplements such as D-sorbitol, D-mannose, D-mannitol, and sodium chloride may be used as an aqueous solution for injection, optionally in combination with a suitable solubilizing agent, for example, alcohol such as ethanol and polyalcohol such as propylene glycol or polyethylene glycol, and a nonionic surfactant such as polysorbate 80™, HCO-50 and the like.

Nonlimiting examples of oily liquid include sesame oil and soybean oil, and it may be combined with benzyl benzoate or benzyl alcohol as a solubilizing agent. Other items that may be included are a buffer such as a phosphate buffer, or sodium acetate buffer, a soothing agent such as procaine hydrochloride, a stabilizer such as benzyl alcohol or phenol, and an antioxidant. The formulated injection can be packaged in a suitable ampule.

In some instances, the level of sialylated glycans (e.g., branched glycans that are sialylated on an α1,3 arm, an α1,6 arm, or both, of the branched glycan in the Fc region, e.g., with a NeuAc-α2,6-Gal terminal linkage) in a preparation of antibodies or Fc-containing polypeptides, produced using a method described herein can be compared to a predetermined or target level (e.g., a level in a reference standard or pharmaceutical specification), e.g., to make a decision regarding the composition of the polypeptide preparation, e.g., a decision to classify, select, accept or discard, release or withhold, process into a drug product, ship, move to a different location, formulate, label, package, release into commerce, or sell or offer for sale the polypeptide, e.g., a recombinant antibody. In other instances, the decision can be to accept, modify or reject a production parameter or parameters used to make the polypeptide, e.g., an antibody. Particular, nonlimiting examples of reference standards include a control level (e.g., a polypeptide produced by a different method) or a range or value in a product specification (e.g., a master batch record, a release specification, an FDA label or Physician's Insert) or quality or identity criterion for a pharmaceutical preparation containing the polypeptide preparation.

In some instances, methods (i.e., evaluation, identification, and production methods) include taking action (e.g., physical action) in response to the methods disclosed herein. For example, a polypeptide preparation is classified, selected, accepted or discarded, released or withheld, processed into a drug product, shipped, moved to a different location, formulated, labeled, packaged, released into commerce, or sold or offered for sale, depending on whether the preselected or target value is met. In some instances, processing may include formulating (e.g., combining with pharmaceutical excipients), packaging (e.g., in a syringe or vial), labeling, or shipping at least a portion of the polypeptide preparation. In some instances, processing includes formulating (e.g., combining with pharmaceutical excipients), packaging (e.g., in a syringe or vial), and labeling at least a portion of the preparation as a drug product described herein. Processing can include directing and/or contracting another party to process as described herein.

In some instances, a biological activity of a polypeptide preparation (e.g., an antibody preparation) is assessed. Biological activity of the preparation can be analyzed by any known method. In some embodiments, a binding activity of a polypeptide is assessed (e.g., binding to a receptor). In some embodiments, a therapeutic activity of a polypeptide is assessed (e.g., an activity of a polypeptide in decreasing severity or symptom of a disease or condition, or in delaying appearance of a symptom of a disease or condition). In some embodiments, a pharmacologic activity of a polypeptide is assessed (e.g., bioavailability, pharmacokinetics, pharmacodynamics). For methods of analyzing bioavailability, pharmacokinetics, and pharmacodynamics of glycoprotein therapeutics, see, e.g., Weiner et al., J. Pharm. Biomed. Anal. 15(5):571-9, 1997; Srinivas et al., J. Pharm. Sci. 85(1):1-4, 1996; and Srinivas et al., Pharm. Res. 14(7):911-6, 1997.

The particular biological activity or therapeutic activity that can be tested will vary depending on the particular polypeptide (e.g., antibody). The potential adverse activity or toxicity (e.g., propensity to cause hypertension, allergic reactions, thrombotic events, seizures, or other adverse events) of polypeptide preparations can be analyzed by any available method. In some embodiments, immunogenicity of a polypeptide preparation is assessed, e.g., by determining whether the preparation elicits an antibody response in a subject.

Route of administration can be parenteral, for example, administration by injection, transnasal administration, transpulmonary administration, or transcutaneous administration. Administration can be systemic or local by intravenous injection, intramuscular injection, intraperitoneal injection, subcutaneous injection.

A suitable means of administration can be selected based on the age and condition of the patient. A single dose of the pharmaceutical composition containing a modified glycoprotein can be selected from a range of 0.001 to 1000 mg/kg of body weight. On the other hand, a dose can be selected in the range of 0.001 to 100000 mg/body weight, but the present disclosure is not limited to such ranges. The dose and method of administration varies depending on the weight, age, condition, and the like of the patient, and can be suitably selected as needed by those skilled in the art.

The materials, methods, and examples are illustrative only and not intended to be limiting. Unless otherwise defined, all technical and scientific terms used herein have the same meaning as commonly understood by one of ordinary skill in the art to which this disclosure belongs. Although methods and materials similar or equivalent to those described herein can be used in the practice or testing of the present disclosure, suitable methods and materials are described herein.

### EXAMPLES

### Example 1 - Galactosylation and sialylation of IVIG

The sialylation of IVIG by the sialyltransferase ST6 was analyzed. IVIG was first galactosylated and then sialylated. The reactions were performed sequentially. There was no purification between galactosylation and sialylation reactions. The relative abundance of glycoforms was analyzed following the sialylation reactions.

### A. Galactosylation

A reaction was set up that contained the following components at the concentrations indicated:

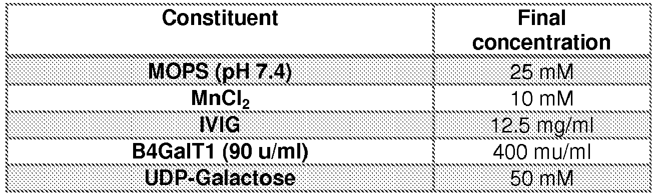

The reaction was incubated for 72 hours at 37 °C.

### B. Sialylation

To an aliquot of the galactosylation reaction were added CMP-NANA, MOPS buffer and ST6Gal1. The final volume was adjusted so that the final concentration of components in the reaction was as indicated.

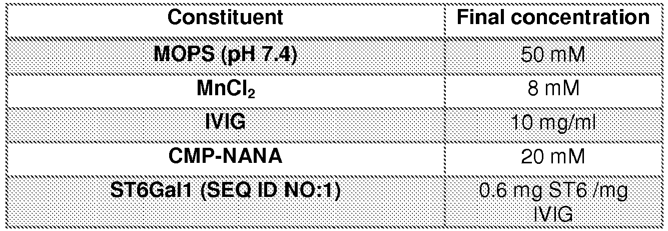

The reaction was incubated at 37 °C. Aliquots were extracted at the times indicated in Figure 2 and frozen at -20 °C for later analyses.

### C. Results

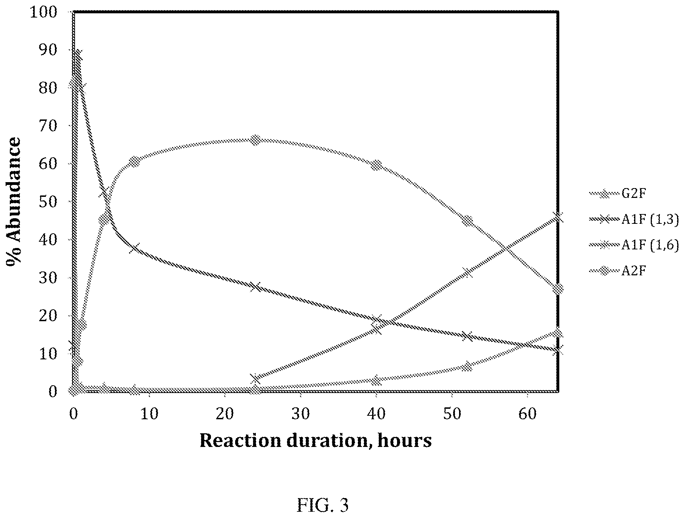

As shown in Figure 3, the predominant glycoform changed over time from G2F to A1F (1,3) to A2F to A1F (1,6). The results are summarized in the reaction scheme depicted in Figure 4. As shown in Figure 4, the product glycoform can change between G2F, A1F (1,3), A2F, and A1F (1,6) during the course of a reaction due to competing addition (forward reaction) and removal (back reaction) steps.

The sialyltransferase ST6 can add sialic acid to either branch of a substrate's biantennary N-glycan. However, these results demonstrate that addition to each branch happens at different rates, resulting in different end products depending on the reaction conditions. Addition of sialic acid to the α1,3 branch is much faster than addition to the α1,6 branch.

These data also demonstrate that sialyltransferase ST6 can also catalyze the removal of sialic acids from N-glycans. The removal of sialic acid from the α1,3 branch is much faster than removal from the α1,6 branch. This can surprisingly lead to the production of Fc glycans substantially or primarily monosialylated on the α1,6 branch by modulating reaction conditions.

This Example demonstrates that reaction conditions can be controlled to produce a glycoprotein product having a predetermined or target sialylation levels. Such conditions can include time, ST6 sialyltransferase concentration, substrate concentration, donor sugar nucleotide concentration, product nucleotide concentration, pH, buffer composition, and/or temperature.

## Claims

1. A method of producing a preparation of glycoproteins comprising Fc regions comprising branched glycans comprising an α1,3 arm and an α1,6 arm, wherein the preparation is an IVIG preparation comprising a target level of disialylated branched glycans having a sialic acid on an α1,3 arm and on an α1,6 arm, wherein the target level of disialylated branched glycans having a sialic acid on an α1,3 arm and on an α1,6 arm is at least 60% of sialylated branched glycans, the method comprising:
providing an IVIG preparation, wherein the IVIG preparation comprises a plurality of glycoproteins comprising Fc regions comprising branched glycans comprising an α1,3 arm and an α1,6 arm; and
contacting the IVIG preparation with an ST6 sialyltransferase and a sialic acid donor under reaction conditions sufficient for the ST6 sialyltransferase substantially to add a sialic acid to an α1,3 arm and to an α1,6 arm of a branched glycan, and not sufficient for the ST6 sialyltransferase substantially to remove a sialic acid from an α1,3 arm of a branched glycan, wherein the ST6 sialyltransferase is a polypeptide involved in the transfer of a sialic acid to a terminal galactose of a glycan through an α2,6 linkage, and wherein the reaction conditions comprise supplementing the sialic acid donor at least once during the reaction;
thereby producing an IVIG preparation having the target level of disialylated branched glycans having a sialic acid on the α1,3 arm and on the α1,6 arm.

2. The method of claim 1, wherein the method further comprises isolating the glycoprotein preparation and optionally further comprises measuring a level of disialylated branched glycans having a sialic acid on an α1,3 arm and on an α1,6 arm.

3. The method of claim 1 or claim 2, wherein the target level of disialylated branched glycans having a sialic acid on an α1,3 arm and on an α1,6 arm is at least 70%, 75%, 80%, 85%, 90%, 95%, or 100% of sialylated branched glycans in the IVIG preparation.

4. The method of claim 1 or claim 2, wherein the target level of disialylated branched glycans having a sialic acid on an α1,3 arm and on an α1,6 arm is at least 75%, 80%, 85%, 90%, 95%, or 100% of sialylated branched glycans in the IVIG preparation.

5. The method of any of claims 1-4, wherein the reaction conditions comprise controlling the reaction kinetics such that: (i) the rate of addition of a sialic acid to an α1,3 arm exceeds the rate of removal of a sialic acid from an α1,3 arm; and (ii) the rate of addition of a sialic acid to an α1,6 arm exceeds the rate of removal of a sialic acid from an α1,6 arm.

6. A method of modulating sialylation of Fc region branched glycans comprising an α1,3 arm and an α1,6 arm in an IVIG preparation, the method comprising:
providing a reaction solution comprising (i) an IVIG preparation comprising Fc region branched glycans comprising an α1,3 arm and an α1,6 arm, (ii) a ST6 sialyltransferase, and (iii) a sialic acid donor; and
incubating the reaction solution under reaction conditions sufficient for the ST6 sialyltransferase to catalyze transfer of a sialic acid to both the α1,3 arm and the α1,6 arm, wherein said step of incubating comprises controlling reaction kinetics such that: (i) the rate of addition of a sialic acid to an α1,3 arm (Rₐ^{1,3}) exceeds the rate of removal of a sialic acid from an α1,3 arm (Rᵣ^{1,3}); and (ii) the rate of addition of a sialic acid to an α1,6 arm (Rₐ^{1,6}) exceeds the rate of removal of a sialic acid from an α1,6 arm (Rᵣ^{1,6}), wherein the ST6 sialyltransferase is a polypeptide involved in the transfer of a sialic acid to a terminal galactose of a glycan through an α2,6 linkage, and wherein the reaction conditions comprise supplementing the sialic acid donor at least once during the reaction;
thereby modulating sialylation of a branched glycan.

7. The method of claim 6, wherein:
(i) controlling reaction kinetics comprises modulating the time of the reaction;
(ii) controlling reaction kinetics comprises modulating the level or activity of the sialyltransferase;
(iii) the method comprises detecting reaction kinetics;
(iv) the method comprises measuring a level of sialylated glycans;
(v) controlling reaction kinetics comprises modulating the rate of removal of a sialic acid from an α1,3 arm (Rᵣ^{1,3}) or the rate of removal of a sialic acid from an α1,6 arm (Rᵣ^{1,6}) by controlling or adjusting the ratio of the sialic acid donor to a sialic acid donor reaction product;
(vi) the sialic acid donor is cytidine 5'-monophospho-N-acetyl neuraminic acid and the sialic acid donor reaction product is cytidine 5'-monophosphate;
(vii) the reaction conditions sufficient for the sialyltransferase to catalyze transfer of the sialic acid to both the α1,3 and α1,6 arms comprises removing a sialic donor reaction product at least once during the reaction; or
(viii) the reaction conditions sufficient for the sialyltransferase to catalyze transfer of the sialic acid to both the α1,3 and α1,6 arms comprises supplementing the sialic donor reaction product at least once during the reaction.

8. A method of producing a drug product comprising a preparation of glycoproteins comprising Fc regions comprising branched glycans comprising an α1,3 arm and an α1,6 arm, wherein the preparation is an IVIG preparation comprising a target level of disialylated branched glycans having a sialic acid on an α1,3 arm and on an α1,6 arm, wherein the target level of disialylated branched glycans having a sialic acid on an α1,3 arm and on an α1,6 arm is at least 60% of sialylated branched glycans, the method comprising:
providing an IVIG preparation, wherein the IVIG preparation comprises a plurality of glycoproteins comprising Fc regions comprising branched glycans comprising an α1,3 arm and an α1,6 arm;
contacting the IVIG preparation with an ST6 sialyltransferase and a sialic acid donor under reaction conditions sufficient for the ST6 sialyltransferase substantially to add a sialic acid to an α1,3 arm and to an α1,6 arm of a branched glycan, and not sufficient for the ST6 sialyltransferase substantially to remove a sialic acid from an α1,3 arm of a branched glycan, thereby producing a preparation of sialylated glycoproteins, wherein the ST6 sialyltransferase is a polypeptide involved in the transfer of a sialic acid to a terminal galactose of a glycan through an α2,6 linkage, and wherein the reaction conditions comprise supplementing the sialic acid donor at least once during the reaction; and
formulating the preparation into a drug product if the preparation meets the target level of disialylated branched glycans having a sialic acid on an α1,3 arm and on an α1,6 arm.

9. The method of claim 8, wherein the method further comprises measuring the level of disialylated branched glycans having a sialic acid on an α1,3 arm and on an α1,6 arm in the preparation.

10. The method of claim 8 or claim 9, wherein the reaction conditions comprise controlling the reaction kinetics such that: (i) the rate of addition of a sialic acid to an α1,3 arm exceeds the rate of removal of a sialic acid from an α1,3 arm; and (ii) the rate of addition of a sialic acid to an α1,6 arm exceeds the rate of removal of a sialic acid from an α1,6 arm.

11. The method of any of claims 8-10, wherein the target level of disialylated branched glycans having a sialic acid on an α1,3 arm and on an α1,6 arm is at least 70%, 75%, 80%, 85%, 90%, 95%, or 100% of sialylated branched glycans in the IVIG preparation.

12. The method of any of claims 8-10, wherein the target level of disialylated branched glycans having a sialic acid on an α1,3 arm and on an α1,6 arm is at least 75%, 80%, 85%, 90%, 95%, or 100% of sialylated branched glycans in the IVIG preparation.

## Patentansprüche

1. Ein Verfahren zum Herstellen eines Präparats von Glykoproteinen, die Fc-Regionen beinhalten, die verzweigte Glykane beinhalten, die einen α1,3-Arm und einen α1,6-Arm beinhalten, wobei das Präparat ein IVIG-Präparat ist, das ein Zielniveau von disialylierten verzweigten Glykanen mit einer Sialinsäure auf einem α1,3-Arm und auf einem α1,6-Arm beinhaltet, wobei das Zielniveau von disialylierten verzweigten Glykanen mit einer Sialinsäure auf einem α1,3-Arm und auf einem α1,6-Arm mindestens 60% der sialylierten verzweigten Glykane beträgt, wobei das Verfahren Folgendes beinhaltet:
Bereitstellen eines IVIG-Präparats, wobei das IVIG-Präparat eine Vielzahl von Glykoproteinen beinhaltet, die Fc-Regionen beinhalten, die verzweigte Glykane beinhalten, die einen α1,3-Arm und einen α1,6-Arm beinhalten; und
In-Kontakt-Bringen des IVIG-Präparats mit einer ST6-Sialyltransferase und einem Sialinsäuredonor unter Reaktionsbedingungen, die dafür ausreichen, dass die ST6-Sialyltransferase im Wesentlichen eine Sialinsäure zu einem α1,3-Arm und zu einem α1,6-Arm eines verzweigten Glykans hinzufügen kann, und nicht dafür ausreichen, dass die ST6-Sialyltransferase im Wesentlichen eine Sialinsäure von einem α1,3-Arm eines verzweigten Glykans entfernen kann, wobei die ST6-Sialyltransferase ein Polypeptid ist, das am Transfer einer Sialinsäure zu einer terminalen Galactose eines Glykans durch eine α2,6-Verknüpfung beteiligt ist, und wobei die Reaktionsbedingungen das mindestens einmalige Ergänzen des Sialinsäuredonors während der Reaktion beinhalten;
somit Herstellen eines IVIG-Präparats, das das Zielniveau von disialylierten verzweigten Glykanen mit einer Sialinsäure auf dem α1,3-Arm und auf dem α1,6-Arm aufweist.

2. Verfahren nach Anspruch 1, wobei das Verfahren ferner das Isolieren des Glykoproteinpräparats beinhaltet und optional ferner das Messen eines Niveaus von disialylierten verzweigten Glykanen mit einer Sialinsäure auf einem α1,3-Arm und auf einem α1,6-Arm beinhaltet.

3. Verfahren nach Anspruch 1 oder Anspruch 2, wobei das Zielniveau von disialylierten verzweigten Glykanen mit einer Sialinsäure auf einem α1,3-Arm und auf einem α1,6-Arm mindestens 70%, 75%, 80%, 85%, 90%, 95% oder 100% der sialylierten verzweigten Glykane in dem IVIG-Präparat beträgt.

4. Verfahren nach Anspruch 1 oder Anspruch 2, wobei das Zielniveau von disialylierten verzweigten Glykanen mit einer Sialinsäure auf einem α1,3-Arm und auf einem α1,6-Arm mindestens 75%, 80%, 85%, 90%, 95% oder 100% der sialylierten verzweigten Glykane in dem IVIG-Präparat beträgt.

5. Verfahren nach einem der Ansprüche 1-4, wobei die Reaktionsbedingungen das Steuern der Reaktionskinetik beinhaltet, sodass: (i) die Rate des Hinzufügens einer Sialinsäure zu einem α1,3-Arm die Rate des Entfernens einer Sialinsäure von einem α1,3-Arm übersteigt; und (ii) die Rate des Hinzufügens einer Sialinsäure zu einem α1,6-Arm die Rate des Entfernens einer Sialinsäure von einem α1,6-Arm übersteigt.

6. Ein Verfahren zum Modulieren der Sialylierung von verzweigten Glykanen der Fc-Region, die einen α1,3-Arm und einen α1,6-Arm beinhalten, in einem IVIG-Präparat, wobei das Verfahren Folgendes beinhaltet:
Bereitstellen einer Reaktionslösung, die Folgendes beinhaltet: (i) ein IVIG-Präparat, das verzweigte Glykane der Fc-Region beinhaltet, die einen α1,3-Arm und einen α1,6-Arm beinhalten, (ii) eine ST6-Sialyltransferase und (iii) einen Sialinsäuredonor; und
Inkubieren der Reaktionslösung unter Reaktionsbedingungen, die dafür ausreichen, dass die ST6-Sialyltransferase den Transfer einer Sialinsäure sowohl zu dem α1,3-Arm als auch dem α1,6-Arm katalysieren kann, wobei der Schritt des Inkubierens das Steuern der Reaktionskinetik beinhaltet, sodass: (i) die Rate des Hinzufügens einer Sialinsäure zu einem α1,3-Arm (Rₐ^{1,3}) die Rate des Entfernen einer Sialinsäure von einem α1,3-Arm (Rᵣ^{1,3}) übersteigt; und (ii) die Rate des Hinzufügens einer Sialinsäure zu einem α1,6-Arm (Rₐ^{1,6}) die Rate des Entfernens einer Sialinsäure von einem α1,6-Arm (Rᵣ^{1,6}) übersteigt, wobei die ST6-Sialyltransferase ein Polypeptid ist, das an dem Transfer einer Sialinsäure zu einer terminalen Galactose eines Glykans durch eine α2,6-Verknüpfung beteiligt ist, und wobei die Reaktionsbedingungen das mindestens einmalige Ergänzen des Sialinsäuredonors während der Reaktion beinhalten;
somit Modulieren der Sialylierung eines verzweigten Glykans.

7. Verfahren nach Anspruch 6, wobei:
(i) das Steuern der Reaktionskinetik das Modulieren der Zeit der Reaktion beinhaltet;
(ii) das Steuern der Reaktionskinetik das Modulieren des Niveaus oder der Aktivität der Sialyltransferase beinhaltet;
(iii) das Verfahren das Nachweisen der Reaktionskinetik beinhaltet;
(iv) das Verfahren das Messen eines Niveaus von sialylierten Glykanen beinhaltet;
(v) das Steuern der Reaktionskinetik das Modulieren der Rate des Entfernens einer Sialinsäure von einem α1,3-Arm (Rᵣ^{1,3}) oder der Rate des Entfernens einer Sialinsäure von einem α1,6-Arm (Rᵣ^{1,6}) durch Steuern oder Anpassen des Verhältnisses des Sialinsäuredonors zu einem Sialinsäuredonor-Reaktionsprodukt beinhaltet;
(vi) der Sialinsäuredonor Cytidin-5'-monophospho-N-acetylneuraminsäure ist und das Sialinsäuredonor Reaktionsprodukt Cytidin-5'-monophosphat ist;
(vii) die Reaktionsbedingungen, die dafür ausreichen, dass die Sialyltransferase den Transfer der Sialinsäure sowohl zu dem α1,3-Arm als auch dem α1,6-Arm katalysieren kann, das mindestens einmalige Entfernen eines Sialinsäuredonor-Reaktionsprodukts während der Reaktion beinhalten; oder
(viii)die Reaktionsbedingungen, die dafür ausreichen, dass die Sialyltransferase den Transfer der Sialinsäure sowohl zu dem α1,3-Arm als auch dem α1,6-Arm katalysieren kann, das mindestens einmalige Ergänzen eines Sialinsäuredonor-Reaktionsprodukts während der Reaktion beinhalten.

8. Ein Verfahren zum Herstellen eines Arzneimittels, das ein Präparat von Glykoproteinen beinhaltet, die Fc-Regionen beinhalten, die verzweigte Glykane beinhalten, die einen α1,3-Arm und einen α1,6-Arm beinhalten, wobei das Präparat ein IVIG-Präparat ist, das ein Zielniveau von disialylierten verzweigten Glykanen mit einer Sialinsäure auf einem α1,3-Arm und auf einem α1,6-Arm beinhaltet, wobei das Zielniveau von disialylierten verzweigten Glykanen mit einer Sialinsäure auf einem α1,3-Arm und auf einem α1,6-Arm mindestens 60% der sialylierten verzweigten Glykane beträgt, wobei das Verfahren Folgendes beinhaltet:
Bereitstellen eines IVIG-Präparats, wobei das IVIG-Präparat eine Vielzahl von Glykoproteinen beinhaltet, die Fc-Regionen beinhalten, die verzweigte Glykane beinhalten, die einen α1,3-Arm und einen α1,6-Arm beinhalten;
In-Kontakt-Bringen des IVIG-Präparats mit einer ST6-Sialyltransferase und einem Sialinsäuredonor unter Reaktionsbedingungen, die dafür ausreichen, dass die ST6-Sialyltransferase im Wesentlichen eine Sialinsäure zu einem α1,3-Arm und zu einem α1,6-Arm eines verzweigten Glykans hinzufügen kann, und die nicht dafür ausreichen, dass die ST6-Sialyltransferase im Wesentlichen eine Sialinsäure von einem α1,3-Arm eines verzweigten Glykans entfernen kann, somit Herstellen eines Präparats von sialylierten Glykoproteinen, wobei die ST6-Sialyltransferase ein Polypeptid ist, das am Transfer einer Sialinsäure zu einer terminalen Galactose eines Glykans durch eine α2,6-Verknüpfung beteiligt ist, und wobei die Reaktionsbedingungen das mindestens einmalige Ergänzen des Sialinsäuredonors während der Reaktion beinhalten; und
Formulieren des Präparats zu einem Arzneimittel, wenn das Präparat das Zielniveau von disialylierten verzweigten Glykanen mit einer Sialinsäure auf einem α1,3-Arm und auf einem α1,6-Arm erreicht.

9. Verfahren nach Anspruch 8, wobei das Verfahren ferner das Messen des Niveaus von disialylierten verzweigten Glykanen mit einer Sialinsäure auf einem α1,3-Arm und auf einem α1,6-Arm in dem Präparat beinhaltet.

10. Verfahren nach Anspruch 8 oder Anspruch 9, wobei die Reaktionsbedingungen das Steuern der Reaktionskinetik beinhaltet, sodass: (i) die Rate des Hinzufügens einer Sialinsäure zu einem α1,3-Arm die Rate des Entfernens einer Sialinsäure von einem α1,3-Arm übersteigt; und (ii) die Rate des Hinzufügens einer Sialinsäure zu einem α1,6-Arm die Rate des Entfernens einer Sialinsäure von einem α1,6-Arm übersteigt.

11. Verfahren nach einem der Ansprüche 8-10, wobei das Zielniveau von disialylierten verzweigten Glykanen mit einer Sialinsäure auf einem α1,3-Arm und auf einem α1,6-Arm mindestens 70%, 75%, 80%, 85%, 90%, 95% oder 100% der sialylierten verzweigten Glykane in dem IVIG-Präparat beträgt.

12. Verfahren nach einem der Ansprüche 8-10, wobei das Zielniveau von disialylierten verzweigten Glykanen mit einer Sialinsäure auf einem α1,3-Arm und auf einem α1,6-Arm mindestens 75%, 80%, 85%, 90%, 95% oder 100% der sialylierten verzweigten Glykane in dem IVIG-Präparat beträgt.

## Revendications

1. Procédé de production d'une préparation de glycoprotéines comprenant des régions Fc comprenant des glycanes ramifiés comprenant un bras α1,3 et un bras α1,6, dans lequel la préparation est une préparation d'Ig IV comprenant un niveau cible de glycanes ramifiés disialylés comportant un acide sialique sur un bras α1,3 et sur un bras α1,6, dans lequel le niveau cible de glycanes ramifiés disialylés comportant un acide sialique sur un bras α1,3 et sur un bras α1,6 est d'au moins 60 % de glycanes ramifiés sialylés, le procédé comprenant :
la fourniture d'une préparation d'Ig IV, la préparation d'Ig IV comprenant une pluralité de glycoprotéines comprenant des régions Fc comprenant des glycanes ramifiés comprenant un bras α1,3 et un bras α1,6 ; et
la mise en contact de la préparation d'Ig IV avec une sialyltransferase ST6 et un donneur d'acide sialique dans des conditions de réaction suffisantes pour que la sialyltransferase ST6 ajoute sensiblement un acide sialique à un bras α1,3 et à un bras α1,6 d'un glycane ramifié, et non suffisantes pour que la sialyltransferase ST6 retire sensiblement un acide sialique d'un bras α1,3 d'un glycane ramifié, la sialyltransferase ST6 étant un polypeptide impliqué dans le transfert d'un acide sialique à un galactose terminal d'un glycane par l'intermédiaire d'une liaison α2,6, et les conditions de réaction comprenant une supplémentation du donneur d'acide sialique au moins une fois durant la réaction ;
en produisant ainsi une préparation d'Ig IV ayant le niveau cible de glycanes ramifiés disialylés comportant un acide sialique sur le bras α1,3 et sur le bras α1,6.

2. Procédé selon la revendication 1, le procédé comprenant en outre une isolation de la préparation de glycoprotéines et comprenant optionnellement en outre la mesure d'un niveau de glycanes ramifiés disialylés comportant un acide sialique sur un bras α1,3 et sur un bras α1,6.

3. Procédé selon la revendication 1 ou la revendication 2, dans lequel le niveau cible de glycanes ramifiés disialylés comportant un acide sialique sur un bras α1,3 et sur un bras α1,6 est d'au moins 70 %, 75 %, 80 %, 85 %, 90 %, 95 %, ou 100 % de glycanes ramifiés sialylés dans la préparation d'Ig IV.

4. Procédé selon la revendication 1 ou la revendication 2, dans lequel le niveau cible de glycanes ramifiés disialylés comportant un acide sialique sur un bras α1,3 et sur un bras α1,6 est d'au moins 75 %, 80 %, 85 %, 90 %, 95 %, ou 100 % de glycanes ramifiés sialylés dans la préparation d'Ig IV.

5. Procédé selon l'une quelconque des revendications 1 à 4, dans lequel les conditions de réaction comprennent un contrôle de la cinétique de réaction de telle sorte que : (i) la vitesse d'addition d'un acide sialique à un bras α1,3 dépasse la vitesse de retrait d'un acide sialique d'un bras α1,3 ; et (ii) la vitesse d'addition d'un acide sialique à un bras α1,6 dépasse la vitesse de retrait d'un acide sialique d'un bras α1,6.

6. Procédé de modulation de la sialylation de glycanes ramifiés de région Fc comprenant un bras α1,3 et un bras α1,6 dans une préparation d'Ig IV, le procédé comprenant :
la fourniture d'une solution de réaction comprenant (i) une préparation d'Ig IV comprenant des glycanes ramifiés de région Fc comprenant un bras α1,3 et un bras α1,6, (ii) une sialyltransferase ST6, et (iii) un donneur d'acide sialique ; et
la mise en incubation de la solution de réaction dans des conditions de réaction suffisantes pour que la sialyltransferase ST6 catalyse le transfert d'un acide sialique à la fois au bras α1,3 et au bras α1,6, ladite étape de mise en incubation comprenant un contrôle de la cinétique de réaction de telle sorte que : (i) la vitesse d'addition d'un acide sialique à un bras α1,3 (Rₐ^{1,3}) dépasse la vitesse de retrait d'un acide sialique d'un bras α1,3 (Rᵣ^{1,3}) ; et (ii) la vitesse d'addition d'un acide sialique à un bras α1,6 (Rₐ^{1,6}) dépasse la vitesse de retrait d'un acide sialique d'un bras α1,6 (Rᵣ^{1,6}), la sialyltransferase ST6 étant un polypeptide impliqué dans le transfert d'un acide sialique à un galactose terminal d'un glycane par l'intermédiaire d'une liaison α2,6, et les conditions de réaction comprenant une supplémentation du donneur d'acide sialique au moins une fois durant la réaction ;
en modulant ainsi la sialylation d'un glycane ramifié.

7. Procédé selon la revendication 6, dans lequel :
(i) le contrôle de la cinétique de réaction comprend une modulation du temps de la réaction ;
(ii) le contrôle de la cinétique de réaction comprend une modulation du niveau ou de l'activité de la sialyltransferase ;
(iii) le procédé comprend une détection de la cinétique de réaction ;
(iv) le procédé comprend la mesure d'un niveau de glycanes sialylés ;
(v) le contrôle de la cinétique de réaction comprend une modulation de la vitesse de retrait d'un acide sialique d'un bras α1,3 (Rᵣ^{1,3}) ou de la vitesse de retrait d'un acide sialique d'un bras α1,6 (Rᵣ^{1,6}) en contrôlant ou en ajustant le rapport du donneur d'acide sialique contre un produit réactionnel du donneur d'acide sialique ;
(vi) le donneur d'acide sialique est l'acide cytidine 5'-monophosphate N-acétylneuraminique et le produit réactionnel du donneur d'acide sialique est la cytidine 5'-monophosphate ;
(vii) les conditions de réaction suffisantes pour que la sialyltransferase catalyse le transfert de l'acide sialique à la fois au bras α1,3 et au bras α1,6 comprennent le retrait d'un produit réactionnel du donneur d'acide sialique au moins une fois durant la réaction ; ou
(viii) les conditions de réaction suffisantes pour que la sialyltransferase catalyse le transfert de l'acide sialique à la fois au bras α1,3 et au bras α1,6 comprennent une supplémentation du produit réactionnel du donneur d'acide sialique au moins une fois durant la réaction.

8. Procédé de production d'un produit médicamenteux comprenant une préparation de glycoprotéines comprenant des régions Fc comprenant des glycanes ramifiés comprenant un bras α1,3 et un bras α1,6, dans lequel la préparation est une préparation d'Ig IV comprenant un niveau cible de glycanes ramifiés disialylés comportant un acide sialique sur un bras α1,3 et sur un bras α1,6, dans lequel le niveau cible de glycanes ramifiés disialylés comportant un acide sialique sur un bras α1,3 et sur un bras α1,6 est d'au moins 60 % de glycanes ramifiés sialylés, le procédé comprenant :
la fourniture d'une préparation d'Ig IV, la préparation d'Ig IV comprenant une pluralité de glycoprotéines comprenant des régions Fc comprenant des glycanes ramifiés comprenant un bras α1,3 et un bras α1,6 ;
une mise en contact de la préparation d'Ig IV avec une sialyltransferase ST6 et un donneur d'acide sialique dans des conditions de réaction suffisantes pour que la sialyltransferase ST6 ajoute sensiblement un acide sialique à un bras α1,3 et à un bras α1,6 d'un glycane ramifié, et non suffisantes pour que la sialyltransferase ST6 retire sensiblement un acide sialique d'un bras α1,3 d'un glycane ramifié, en produisant ainsi une préparation de glycoprotéines sialylées, la sialyltransferase ST6 étant un polypeptide impliqué dans le transfert d'un acide sialique à un galactose terminal d'un glycane par l'intermédiaire d'une liaison α2,6, et les conditions de réaction comprenant une supplémentation du donneur d'acide sialique au moins une fois durant la réaction ; et
une formulation de la préparation en un produit médicamenteux si la préparation atteint le niveau cible de glycanes ramifiés disialylés comportant un acide sialique sur un bras α1,3 et sur un bras α1,6.

9. Procédé selon la revendication 8, le procédé comprenant en outre une mesure du niveau de glycanes ramifiés disialylés comportant un acide sialique sur un bras α1,3 et sur un bras α1,6 dans la préparation.

10. Procédé selon la revendication 8 ou la revendication 9, dans lequel les conditions de réaction comprennent un contrôle de la cinétique de réaction de telle sorte que : (i) la vitesse d'addition d'un acide sialique à un bras α1,3 dépasse la vitesse de retrait d'un acide sialique d'un bras α1,3 ; et (ii) la vitesse d'addition d'un acide sialique à un bras α1,6 dépasse la vitesse de retrait d'un acide sialique d'un bras α1,6.

11. Procédé selon l'une quelconque des revendications 8 à 10, dans lequel le niveau cible de glycanes ramifiés disialylés comportant un acide sialique sur un bras α1,3 et sur un bras α1,6 est d'au moins 70 %, 75 %, 80 %, 85 %, 90 %, 95 %, ou 100 % de glycanes ramifiés sialylés dans la préparation d'Ig IV.

12. Procédé selon l'une quelconque des revendications 8 à 10, dans lequel le niveau cible de glycanes ramifiés disialylés comportant un acide sialique sur un bras α1,3 et sur un bras α1,6 est d'au moins 75 %, 80 %, 85 %, 90 %, 95 %, ou 100 % de glycanes ramifiés sialylés dans la préparation d'Ig IV.
